# EUROPEAN PATENT SPECIFICATION

(11) **EP 1 861 718 B1**
(45) Date of publication and mention of the grant of the patent: **05.05.2010**
(21) Application number: 06724890.6
(22) Date of filing: 27.02.2006
(51) Int. Cl.: G01N 33/74

(54) **USE OF XCRI FOR DIAGNOSING OR MONITORING OF IMMUNE TOLERANCE**
VERWENDUNG VON XCRI ZUR DIAGNOSE ODER ÜBERWACHUNG VON IMMUNTOLERANZ
UTILISATION DE XCR1 POUR LE DIAGNOSTIC ET SURVEILLANCE DE LA TOLÉRANCE IMMUNITAIRE

(30) Priority: 01.03.2005 EP 05004392
(43) Date of publication of application: 05.12.2007
(73) Proprietor: DKFZ Deutsches Krebsforschungszentrum, Stiftung des öffentlichen Rechts, 69120 Heidelberg (DE); Ruprecht-Karls-Universität Heidelberg, 69117 Heidelberg (DE)
(72) Inventor: OELERT, Thilo, 69121 Heidelberg (DE); ARNOLD, Bernd, 69124 Heidelberg (DE); HÄMMERLING, Günter, 69118 Heidelberg (DE)
(74) Representative: Dick, Alexander
(86) International application number: PCT/EP2006/060285
(87) International publication number: WO 2006/092385

(56) References cited:
- WO-A-01/72830
- WO-A-02/12343
- WO-A-02/059608
- US-A1- 2003 124 627
- US-A1- 2004 191 255
- HIPKIN R WILLIAM ET AL: "Cloning and pharmacological characterization of CXCR1 and CXCR2 from Macaca fascicularis" JOURNAL OF PHARMACOLOGY AND EXPERIMENTAL THERAPEUTICS, vol. 310, no. 1, 1 July 2004 (2004-07-01), pages 291-300, XP002335821 ISSN: 0022-3565
- MIDDEL PETER ET AL: "Expression of the T-cell chemoattractant chemokine lymphotactin in Crohn's disease" AMERICAN JOURNAL OF PATHOLOGY, vol. 159, no. 5, November 2001 (2001-11), pages 1751-1761, XP002335820 ISSN: 0002-9440
- BURST VOLKER R ET AL: "Urinary chemokine (CK) and chemokine receptor (CKR) expression in renal transplantation" JOURNAL OF THE AMERICAN SOCIETY OF NEPHROLOGY, vol. 12, no. Program and Abstract Issue, September 2001 (2001-09), page 854A, XP008049762 & ASN (AMERICAN SOCIETY OF NEPHROLOGY)/ISN (INTERNATIONAL SOCIETY OF NEPHROLOGY) WORLD CONGRESS OF NEP; SAN FRANCISCO, CA, USA; OCTOBER 10-17, 2001 ISSN: 1046-6673

## Description

The present invention relates to diagnosing and/or monitoring of immune tolerance, particularly in the context of T cell-mediated immunity, more particularly in the context of transplantation.

Transplantation is the only possible treatment option in many types of diseases, particularly in cases of end-stage organ failure. Most transplanted organs are allotransplants, i.e. they are derived from donors who are genetically not identical to the recipients. Without medication, these transplants are quickly recognized and destroyed by the immune system of the recipient. Responsible for this rejection reaction are mainly the T cells, which are immune cells that attack and destroy any cells that they recognize as being non-self or "foreign". The recognition of non-self or foreign cells is achieved by the recognition of so-called foreign antigens, i.e. molecules e.g. peptides or processed peptides, on the cell that is being recognized and attacked.

A major breakthrough in transplantation medicine has been the introduction of powerful immunosuppressant drugs, such as cyclosporine A. The immunosuppressants tame the immune system of the recipient and thus allow the transplant to survive. However, prolonged treatment with immunosuppressants causes severe side-effects, e.g. a dramatic increase in susceptibility to cancer, lethal infections, diabetes, depressions and blood pressure increase. Thus, although most patients do now survive for the first year after transplantation, long-term survival has not improved. In fact, more than 50 % of the patients die within 5-15 years after transplantation, frequently from side-effects of the immunosuppressants, a fact that has been termed "death with functioning organ".

An alternative to high dosage of immunosuppressants is induction of immunologic tolerance to the transplant: several patients have been reported to have survived without immunosuppressants for many years. In other patients, the dosage of immunosuppressant could be significantly reduced. It appears that the immune system of such patients has become tolerant to the foreign organ. More recently, treatment regimens have been devised to actively induce tolerance in transplant patients. According to these treatment protocols, the T cells are induced to become "tolerant" and do not attack the foreign organ any more.

Promising as such an approach may be, it has not entered into regular clinical practice, as it is impossible to predict whether a patient's T cells will become tolerant or not. Reducing the dosage of immunosuppressant is associated with a high risk, as it may trigger an uncontrollable rejection reaction. So far, no method has been reported that allows to monitor or diagnose whether the immune system has become tolerant to the donated organ.

Mazariegos et al. have conducted a small study in children who received liver transplants (Mazariegos, G.V., Reyes, J., Webber, S.A. et al. (2004). Cytokine gene polymorphisms in children successfully withdrawn from immunosuppression after liver transplantation. Transplantation, vol. 73 (8), pp. 1342-1345). According to the authors, children successfully withdrawn from immunosuppression have a genetic predisposition towards low TNFα and high/intermediate IL-10. However, the study merely relates to a genetic predisposition of patients likely to become tolerant.

In a different study, the same authors described that the ratio of certain subsets of dendritic cells in peripheral blood correlates with successful withdrawal of immunosuppression in liver transplant patients (Mazariegos, G.V., Zahorchak, A.F., Reyes, J., et al. (2003). Dendritic cell subset ratio in peripheral blood correlates with successful withdrawal of immunosuppression in liver transplant patients. American Journal of Transplantation, vol. 3 (6), pp. 689-96). However, the clinical relevance of this correlation remains unclear. Various chemokine receptors and their ligands have been suggested as markers to diagnose organ rejections (WO 01/72830). Their role in the tolerance status of a subject remains unknown. Therefore, the problem underlying the invention is the continuing and urgent need to provide a direct marker for tolerance of the immune system to a received transplant.

The problem is solved by a method for measuring the tolerance status of a T cell sample, comprising the step of measuring the expression level of XCR1 in a T cell sample.

Especially, the invention relates to a method for measuring the tolerance status of a T cell sample, comprising the steps of
(a) measuring the expression level of XCR1 in a T cell sample,
(b) comparing the measured expression level with a reference value, said reference value being representative of the expression level of XCR1 in a control T cell sample.

In the context of the present invention, it has been found that the expression level of XCR1 in a T cell or T cell sample correlates with the tolerance status of the T cell, T cell sample, and the immune system of a subject harboring such T cells. According to the invention, an increase in the expression level of XCR1 in a T cell indicates that the T cell, sample, or immune system has become more tolerant. On the other hand, a decrease in the expression level of XCR1 indicates that tolerance was not established in these cells.

Advantageously, the present invention is supported by findings in vivo. The actual physiological mechanisms leading to tolerance appear to be manifold and are not fully understood. However, the present invention has proven to work in subjects showing functional tolerance (see Example 2). Thus, the present invention works in the context of functional tolerance and is not merely based on a particular hypothesis or cellular mechanism of tolerance.

XCR1 (GPR5) is a receptor for Single C Motif-1/Lymphotactin. Single C motif-1 (SCM-1)/lymphotactin is a member of the chemokine superfamily, but retains only the 2nd and 4th of the four cysteine residues conserved in other chemokines. In humans, two highly homologous genes are present, SCM-1α and SCM-1β. XCR1 mediates chemotactic responses and calcium mobilization to SCM-1α and SCM-1β in murine L1.2 cells (Yoshida, T., Imai, T., Kakizaki, M., et al. (1998). Identification of Single C Motif-1/Lymphotactin Receptor XCR1). SCM-1α binds to human XCR1 with high affinity (Kd of 10 nM) (see e.g. Yoshida, T., Imai, T., et al. (1998), cited above). XCR1 is presumed to be a G protein coupled receptor. Human XCR1 is espressed strongly in placenta and weakly in spleen and thymus.

Similar to the human orthologue, murine pre-B cells expressing mXCR1 responded to mSCM-1 in chemotactic and calcium mobilization assays (Yoshida, T., Izawa, D., Nakayama, T., et al. (1999). Molecular cloning of mXCR1, the murine SCM-1/lymphotactin receptor. FEBS letters vol. 458, 37-40). mXCR1 RNA was weakly expressed in spleen and lung of normal C57BL/6 mice. In spleen, CD8+ cells and NK1.1+ cells wer found to express mXCR1 (Yoshida, T., Izawa, D., Nakayama, T., et al. (1999), cited above).

The term T cell (also known as T lymphocyte) is known to the person skilled in the art. T cells constitute a major part of the lymphocytes. Lymphocytes are small mononuclear white blood cells present in large numbers in lymphoid tissues and circulating in blood and lymphe. Another type of lymphocytes are the B cells or B-lymphocytes, which may interact with the T cells. T cells are lymphocytes originating in the thymus (in mammals) and are present in secondary lymphoid tissues (e.g. lymph nodes, spleen) and blood. T cells are involved in cellular immune reactions, i.e. they attack cells expressing particular antigens on their sufaces. However, T cells also aid the production of antibodies. T cells bear antigen-specific receptors (T cell receptors) on their surface and only react to an antigen presented to them on the surface of a cell. Each T cell bears only one particular type of T cell receptor. Thus, the T cell reacts only if an antigen is specifically bound or "recognized" by the T cell receptor.

The main types of T cells are (1) cytotoxic T cells, which recognize and kill body cells that have become antigenically altered in some way (e.g. by infection with an intracellular pathogen, e.g. a virus), (2) helper T cells, which are activated by foreign antigen on the surface of antigen presenting cells antigen-presenting cells (APC's, e.g. dendritic cells) and in turn activate the appropriate B cells, and (3) regulatory T cells (particularly in older literature also described as "suppressor T cells"), which are involved in suppressing or regulating immune responses and the general regulation of the immune system.

The term T cell sample relates to any sample comprising at least one T cell, preferably isolated from other cell types.

The term tolerance relates to immune tolerance or immunological tolerance. These terms are well-known to the person skilled in the art. Tolerance can be understood on the level of a subject or its immune system, as well as on the level of cell populations or cells.

A subject according to the present invention may be an animal or a human being. Preferably, the animal is a mammal, particularly a rodent or a non-human primate. For example, the animal may be a fish (e.g. zebrafish or medaka), frog (e.g. *Xenopus leavis*), chicken, mouse, rat, guinea pig, rabbit, cat, dog, pig, monkey, or ape. Preferably, the human being is a patient according to the present invention.

On the level of a subject, and in the context of transplantation, tolerance relates to a lack of of acute and chronic rejection and indefinite transplant survival with normal transplant function in an immunocompetent subject. Particularly, tolerance relates to a lack of chronic rejection.

It is evident that in the context of autoimmunity, tolerance is understood to relate to the lack of acute and chronic autoimmune reaction against tissue or a cell of the own body ("self" tissue or cells) in an immunocompetent subject. Particularly, tolerance relates to the absence of chronic autoimmune reaction.

Analogously, in the context of allergies, tolerance is understood to relate to the lack of acute and chronic allergic reaction against a relevant antigen in an immunocompetent subject. Particularly, tolerance relates to the absence of chronic allergic reaction.

Tolerance does not mean complete unresponsiveness toward the transplant, "self" tissue or cell, or allergen. Rather tolerance means a lack of destructive immune response towards it in the presence of general immune competence.

According to the present invention, immune competence is the ability of a subject or its immune system to mount a normal or almost normal immune response against an antigen, particularly against other antigens than a transplant, "self" tissue or cell, or allergen. In contrast, there is usually an deficiency of immune competence in subjects treated with immunosuppressants. The degree of deficiency will typically be correlated with the dosage of immunosuppressant administered.

It should be understood that there may be different levels of tolerance, reflecting a "tolerance status". For example, the tolerance status may range from complete unresponsiveness (high tolerance) via a clinically irrelevant immune response (intermediate tolerance) to a presence but clinically acceptable degree of destructive immune response (low tolerance).

Analogously, in the context of allergies, the tolerance status may range from complete unresponsiveness (high tolerance or absence of a allergic reaction) via present but clinically irrelevant allergic reaction (intermediate tolerance) to present but clinically acceptable level of allergic reaction (low tolerance).

The term tolerance may also be understood on the level of single T cells. In this context, it should be noted that T cells according to the present invention can be functionally distinguished into three major groups: (1) naive T cells, (2) activated T cells, and (3) tolerant T cells.

A naive T cell has not been in contact with its antigen, but will become activated upon contact and will attack the cell expressing the antigen on its surface.

An activated T cell has been in contact with its antigen and will attack a cell expressing the antigen on its surface.

A tolerant T cell has been in contact with the antigen but will not attack. Particularly, a T cell differentiating into a regulatory T cell or acquiring the properties of a regulatory T cell will be tolerant or induce tolerance according to the present invention.

Different mechanisms of tolerance have been discussed, including deletion (e.g. deletion of auto-reactive cells), anergy, immunological ignorance, and immune deviation.

Anergy relates to the hyporesponsive state induced in T cells, which had contact with the antigen, but not full co-stimulation (in addition to contact with the antigen, co-stimulation ( e.g. through co-stimulatory proteins such as the B1 protein) is required to fully activate a T cell).

Immunological ignorance relates to a phenomenon in which B or T cells coexist with the antigen without being affected by it.

Immune deviation relates to the switching of a T_{H}1 to T_{H}2 response. The T_{H}2 response is less damaging to the tissue than the T_{H}1 response.

The different mechanisms of tolerance described above may not be entirely distinct, but may be linked by similar molecular mechanisms. An important role in tolerance seem to play the so-called regulatory T cells. These are T cells, which are able to suppress or regulate immune responses of other T cells. Therefore, the term tolerance can also be understood on the level of a population of T cells, e.g. a T cell population containing potentially autoreactive T cells, whose reactivity against their antigens is suppressed by regulatory T cells.

According to the present invention, measurement of the expression level of XCR1 allows to determine the tolerance status of subjects, T cells, or T cell samples.

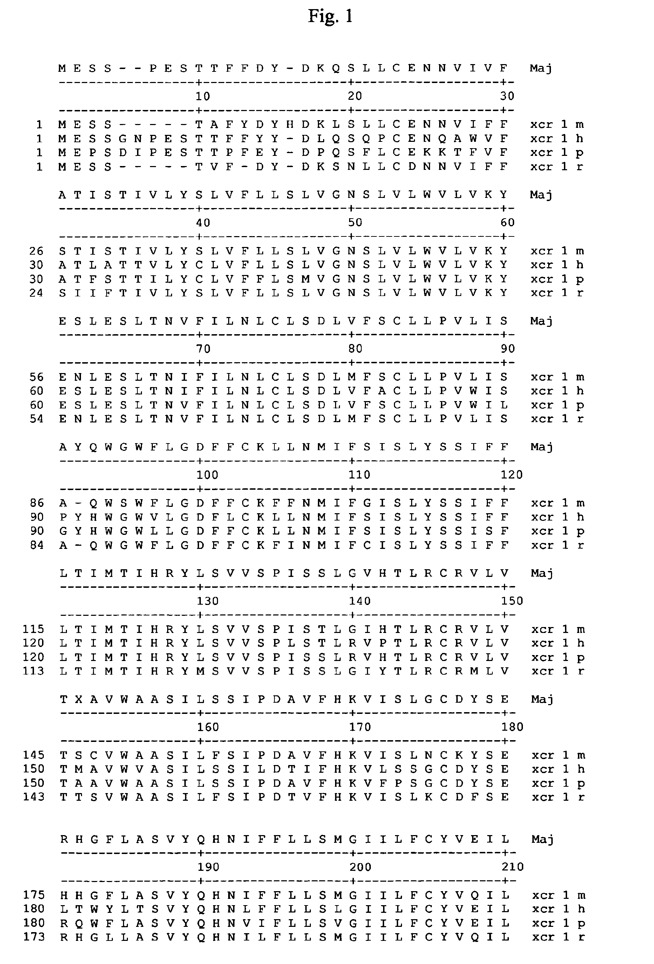

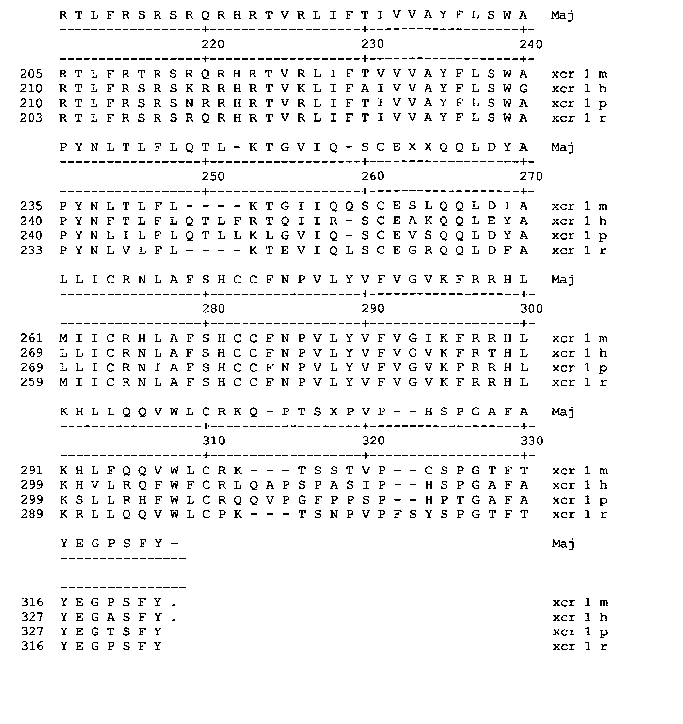

According to the present invention, the term XCR1 relates to human XCR1 (GenBank Acc. No. AY275469, see Fig. 1) or any ortholog thereof in a vertebrate, particular mammal. Examples for vertebrates include teleosts and other fish (particularly zebrafish and medaka), amphibia (e.g. *Xenopus leavis*), birds (e.g. chicken), and mammals. Examples for mammals include mouse, rat, rabbit, guinea pig, pig, cat, dog, and primates (e.g. monkeys and apes). Murine, porcine, and rat orthologs of mXCR1 are shown in Fig.
1. The term ortholog is known to the person skilled in the art. An ortholog is essentially the same gene or protein as the one it is compared to, but it is preferably derived from another species (an ortholog's sequence similarity originates preferably from a speciation event rather than a duplication event).

It is known to a person skilled in the art that orthologs are very likely to serve the same or similar biological function as their counterparts in another species. In the present case, the relevant biological function is the correlation of the expression level with the tolerance or activation status of a T cell. Orthologs can be identified based on sequence similarity to the sequences described herein, e.g. through database searches or hybridization experiments as familiar to the person skilled in the art.

Suitable experimental conditions for determining whether a given DNA or RNA sequence "hybridizes" to a specified polynucleotide or oligonucleotide probe involve presoaking of the filter containing the DNA or RNA to examine for hybridization in 5 x SSC (sodium chloride/sodium citrate) buffer for 10 minutes, and prehybridization of the filter in a solution of 5 x SSC, 5 x Denhardt's solution, 0,5 % SDS and 100 mg/ml of denaturated sonicated salmon sperm DNA (Maniatis et al. (1989), Molecular cloning: A laboratory manual, Cold Spring Harbor Lab., Cold Spring Harbor, NY), followed by hybridization in the same solution containing a concentration of 10 ng/ml of a random primed (Feinberg, A.P. and Vogelstein, B. (1983), Anal. Biochem. 132:6-13), ³²P-dCTP-labeled (specific activity > 1 x 10⁹ cpm/µg) probe for 12 hours at approximately 45°C. The filter is then washed twice for 30 minutes in 2 x SSC, 0,5% SDS at at least 55°C (low stringency), at least 60°C (medium stringency), preferably at least 65°C (medium/high stringency), more preferably at least 70°C (high stringency) or most preferably at least 75°C (very high stringency). The hybridized probe can be detected by methods known in the art, e.g. by use of x-ray film or a "phosphor imager".

Typically, an ortholog will have a sequence identity based on the amino acid sequence of at least 50%, particularly 60%, more particularly 65%, more particularly 70%, most particularly 75%.

Examples for orthologs of XCR1 are disclosed in Fig. 1. Using the DNASTAR MegAlign software (GATC Biotech AG, Konstanz, Germany; Martinez/Needle-man method), it was found that the similarity index of the cDNA sequences of mouse and human XCR1 (Genebank designation numbers NM_011798 and AY275469) was 69.7 % with minor gaps. No splice variants are described for the mouse or human sequences. Further XCR1 cDNA and protein sequences are known for rat and pig (Genebank designation numbers XP_236740 and NM_001001622).

The higher the sequence similarity, the higher is the probability that the orthologs will serve the same or similar biological function. Similarity of the biological function can be confirmed by comparing the properties of the ortholog with those of human XCR1 or murine mXCR1. For comparison, the entirety of assays and properties (e.g. expression patterns) disclosed in this specification and the literature cited herein may be used and interpreted as deemed appropriate by the person skilled in the art. In particular, expression of in CD4+CD25+ T cells and/or increased expression in tolerant T cells (as compared to naive or activated T cells) are properties indicating that the analyzed gene serves the same or similar biological function and that it represents an ortholog of XCR1.

The term XCR1 relates both to the XCR1 gene(s) as well as the XCR1 gene products, particularly messenger RNAs and proteins.

The term XCR1 is also intended to include naturally occurring variants. Examples for naturally occurring variants are alleles of XCR1, which may be present in the human or animal population, as well as splice variants or post-translational modifications, such as phosphorylations, glycosylations, and the like. Also naturally occuring degradation products or fragments are considered to be included as long as the are specific, i.e. as long as they can be assigned to the intact molecule they were derived from. Such specific fragments typically have a length of more than 15, particularly more than 20 base pairs (in the case of nucleic acids) or a length of more than 6, particularly more than 10 amino acids.

Unless otherwise specified, the manipulations of nucleic acids, peptides, polypeptides or proteins can be performed using standard methods of molecular biology and immunology (see, e.g. Maniatis et al. (1989), Molecular cloning: A laboratory manual, Cold Spring Harbor Lab., Cold Spring Harbor, NY; Ausubel, F.M. et al. (eds.) "Current protocols in Molecular Biology". John Wiley and Sons, 1995; Tijssen, P., Practice and Theory of Enzyme Immunoassays, Elsevier Press, Amsterdam, Oxford, New York, 1985).

The expression level of XCR1 can be determined at the level of the protein as well as the transcript of XCR1. In this context, the term "measuring" relates preferably to a quantitative or semi-quantitative determination of the expression level.

The expression level can be measured by measuring the amount or the concentration of XCR1. Preferably, the expression level is determined as the concentration in a given T cell or T cell sample (e.g. the amount of XCR1 per cell or sample, or the concentration of XCR1 in the cell or sample). For the purpose of the invention, it may not be necessary to measure the absolute expression level. Usually, it is sufficient to measure the relative expression level compared to the expression level in an appropriate control. Measurement can also be carried out by measuring derivatives or fragments specific for XCR1, such as specific fragments contained in nucleic acid or protein digests.

Measurement of XCR1 nucleic acids, particularly mRNA, can be performed according to any method known and considered appropriate by the person skilled in the art.

Examples for measurement of RNA include Northern hybridization, RNAse protection assays, in situ hybridization, and aptamers, e.g. Sephadex-binding RNA ligands (Srisawat, C., Goldstein I.J., and Engelke, D.R. (2001). Sephadex-binding RNA ligands: rapid affinity purification of RNA from complex RNA mixtures. Nucleic Acids Research, vol. 29, no. 2 e4).

Furthermore, RNA can be reversely transcribed to cDNA. Therefore methods for measurement of DNA can be employed for measurement of RNA as well, e.g. Southern hybridization, polymerase chain reaction (PCR), Ligase chain reaction (LCR) (see e.g. Cao, W. (2004) Recent developments in ligase-mediated amplification and detection. Trends in Biotechnology, vol. 22 (1), p. 38-44), RT-PCR, real time RT-PCR, quantitative RT-PCR, and microarray hybridization (see e.g. Frey, B., Brehm, U., and Kübler, G., et al. (2002). Gene expression arrays: highly sensitive detection of expression patterns with improved tools for target amplification. Biochemica, vol. 2, p. 27-29).

Measurement of DNA and RNA may also be performed in solution, e.g. using molecular beacons, peptide nucleic acids (PNA), or locked nucleic acids (LNA) (see e.g. Demidov, V.V. (2003). PNA and LNA throw light on DNA. Trends in Biotechnology, vol. 21(1), p. 4-6).

Measurement of XCR1 protein or protein fragments can be carried out according to any method known for measurement of peptides or polypeptides of interest. The person skilled in the art is able to choose an appropriate method.

The person skilled in the art is familiar with different methods of measuring the level of a peptide or polypeptide. The term "level" relates to amount or concentration of a peptide or polypeptide in the sample.

Measuring can be done directly or indirectly. Indirect measuring includes measuring of cellular responses, bound ligands, labels, or enzymatic reaction products.

Measuring can be done according to any method known in the art, such as cellular assays, enzymatic assays, or assays based on binding of ligands. Typical methods are described in the following.

In one embodiment, the method for measuring the level of a peptide or polypeptide of interest comprises the steps of (a) contacting the peptide or polypeptide with a suitable substrate for an adequate period of time, (b) measuring the amount of product.

In another embodiment, the method for measuring the level of a peptide or polypeptide of interest comprises the steps of (a) contacting the peptide or polypeptide with a specifically binding ligand, (b) (optionally) removing non-bound ligand, (c) measuring the amount of bound ligand.

In another embodiment, the method for measuring the level of a peptide or polypeptide of interest comprises the steps of (a) (optionally) fragmenting the peptides or polypeptides of a sample, (b) (optionally) separating the peptides or polypeptides or fragments thereof according to one or more biochemical or biophysical properties (e.g. according to binding to a solid surface or their run-time in a chromatographic setup), (c) determining the amount of one or more of the peptides, polypeptides, or fragments, (d) determining the identity of one or more of the peptides, polypeptides or fragments of step (c) by mass spectrometry. An overview of mass spectrometric methods is given e.g. by Richard D. Smith (2002). Trends in mass spectrometry instrumentation for proteomics. Trends in Biotechnology, Vol. 20, No. 12 (Suppl.), pp. S3-S7).

Other typical methods for measurement include measuring the amount of a ligand binding specifically to the peptide or polypeptide of interest. Binding includes both covalent and non-covalent binding.

A ligand can be any peptide, polypeptide, nucleic acid, or other substance binding to the peptide or polypeptide of interest. It is well known that peptides or polypeptides, if obtained or purified from the human or animal body, can be modified, e.g. by glycosylation. A suitable ligand may bind the peptide or polypeptide also via such sites.

Preferably, the ligand should bind specifically to the peptide or polypeptide to be measured. "Specific binding" means that the ligand should not bind substantially to ("cross-react" with) another peptide, polypeptide or substance present in the sample investigated. Preferably, the specifically bound protein or isoform should be bound with at least 3 times higher, more preferably at least 10 times higher and even more preferably at least 50 times higher affinity than any other relevant peptide or polypeptide.

Non-specific binding may be tolerable, particularly if the investigated peptide or polypeptide can still be distinguished and measured unequivocally, e.g. by separation according to its size (e.g. by electrophoresis), or by its relatively higher abundance in the sample.

Binding of the ligand can be measured by any method known in the art. Preferably, the method is semi-quantitative or quantitative. Suitable methods are described in the following.

First, binding of a ligand may be measured directly, e.g. by NMR or surface plasmon resonance.

Second, if the ligand also serves as a substrate of an enzymatic activity of the peptide or polypeptide of interest, an enzymatic reaction product may be measured (e.g. the amount of a protease can be measured by measuring the amount of cleaved substrate, e.g. on a Western Blot). For measurement of enzymatic reaction products, preferably the amount of substrate is saturating. The substrate may also be labeled with a detectable lable prior to the reaction. Preferably, the sample is contacted with the substrate for an adequate period of time. An adequate period of time refers to the time necessary for an detectable, preferably measurable amount of product to be produced. Instead of measuring the amount of product, the time necessary for appearance of a given (e.g. detectable) amount of product can be measured.

Third, the ligand may be coupled covalently or non-covalently to a label allowing detection and measurement of the ligand. Labeling may be done by direct or indirect methods. Direct labeling involves coupling of the label directly (covalently or non-covalently) to the ligand. Indirect labeling involves binding (covalently or non-covalently) of a secondary ligand to the first ligand. The secondary ligand should specifically bind to the first ligand. Said secondary ligand may be coupled with a suitable label and/or be the target (receptor) of tertiary ligand binding to the secondary ligand. The use of secondary, tertiary or even higher order ligands is often used to increase the signal. Suitable secondary and higher order ligands may include antibodies, secondary antibodies, and the well-known streptavidin-biotin system (Vector Laboratories, Inc.)

The ligand or substrate may also be "tagged" with one or more tags as known in the art. Such tags may then be targets for higher order ligands. Suitable tags include biotin, digoxygenin, His-Tag, Glutathion-S-Transferase, FLAG, GFP, myc-tag, influenza A virus haemagglutinin (HA), maltose binding protein, and the like. In the case of a peptide or polypeptide, the tag is preferably at the N-terminus and/or C-terminus.

Suitable labels are any labels detectable by an appropriate detection method. Typical labels include gold particles, latex beads, acridan ester, luminol, ruthenium, enzymatically active labels, radioactive labels, magnetic labels ("e.g. magnetic beads", including paramagnetic and superparamagnetic labels), and fluorescent labels.

Enzymatically active labels include e.g. horseradish peroxidase, alkaline phosphatase, beta-Galactosidase, Luciferase, and derivatives thereof Suitable substrates for detection include di-amino-benzidine (DAB), 3,3'-5,5'-tetramethylbenzidine, NBT-BCIP (4-nitro blue tetrazolium chloride and 5-bromo-4-chloro-3-indolyl-phosphate, available as ready-made stock solution from Roche Diagnostics), CDP-Star™ (Amersham Biosciences), ECF™ (Amersham Biosciences). A suitable enzyme-substrate combination may result in a colored reaction product, fluorescence or chemoluminescence, which can be measured according to methods known in the art (e.g. using a light-sensitive film or a suitable camera system). As for measuring the enyzmatic reaction, the criteria given above apply analogously.

Typical fluorescent labels include fluorescent proteins (such as GFP and its derivatives), Cy3, Cy5, Texas Red, Fluorescein, and the Alexa dyes (e.g. Alexa 568). Further fluorescent labels are available e.g. from Molcular Probes (Oregon). Also the use of quantum dots as fluorescent labels is contemplated.

Typical radioactive labels include 35S, 125I, 32P, 33P and the like. A radioactive label can be detected by any method known and appropriate, e.g. a light-sensitive film or a phosphor imager.

Suitable measurement methods according the present invention also include precipitation (particularly immunoprecipitation), electrochemiluminescence (electro-generated chemiluminescence), RIA (radioimmunoassay), ELISA (enzyme-linked immunosorbent assay), sandwich enzyme immune tests, electrochemiluminescence sandwich immunoassays (ECLIA), dissociation-enhanced lanthanide fluoro immuno assay (DELFIA), scintillation proximity assay (SPA), turbidimetry, nephelometry, latex-enhanced turbidimetry or nephelometry, solid phase immune tests, and mass spectrometry such as SELDI-TOF, MALDI-TOF, or capillary electrophoresis-mass spectrometry (CE-MS). Further methods known in the art (such as gel electrophoresis, 2D gel electrophoresis, SDS polyacrylamid gel electrophoresis (SDS-PAGE), Western Blotting), can be used alone or in combination with labeling or other dectection methods as described above.

Furthermore, suitable methods include microplate ELISA-based methods, fully-automated or robotic immunoassays (available for example on ElecsysTM analyzers), CBA (an enzymatic Cobalt Binding Assay, available for example on Roche-HitachiTM analyzers), and latex agglutination assays (available for example on Roche-HitachiTM analyzers).

Preferred ligands include antibodies, nucleic acids, peptides or polypeptides, and aptamers, e.g. nucleic acid or peptide aptamers. Methods to such ligands are well-known in the art. For example, identification and production of suitable antibodies or aptamers is also offered by commercial suppliers. The person skilled in the art is familiar with methods to develop derivatives of such ligands with higher affinity or specificity. For example, random mutations can be introduced into the nucleic acids, peptides or polypeptides. These derivatives can then be tested for binding according to screening procedures known in the art, e.g. phage display.

The term "antibody" as used herein includes both polyclonal and monoclonal antibodies, as well as fragments thereof, such as Fv, Fab and F(ab)2 fragments that are capable of binding antigen or hapten.

In another preferred embodiment, the ligand, preferably chosen from the group consisting of nucleic acids, peptides, polypeptides, more preferably from the group consisting of nucleic acids, antibodies, or aptamers, is present on an array.

Said array contains at least one additional ligand, which may be directed against a peptide, polypeptide or a nucleic acid of interest. Said additional ligand may also be directed against a peptide, polypeptide or a nucleic acid of no particular interest in the context of the present invention. Preferably, ligands for at least three, preferably at least five, more preferably at least eight peptides or polypeptides of interest in the context of the present invention are contained on the array.

Binding of the ligand on the array may be detected by any known readout or detection method, e.g. methods involving optical (e.g. fluorescent), electrochemical, or magnetic signals, or surface plasmon resonance.

According to the present invention, the term "array" refers to a solid-phase or gel-like carrier upon which at least two compounds are attached or bound in one-, two- or three-dimensional arrangement. Such arrays (including "gene chips", "protein chips", antibody arrays and the like) are generally known to the person skilled in the art and typically generated on glass microscope slides, specially coated glass slides such as polycation-, nitrocellulose- or biotin-coated slides, cover slips, and membranes such as, for example, membranes based on nitrocellulose or nylon. The array may include a bound ligand or at least two cells expressing each at least one ligand.

It is also contemplated to use "suspension arrays" as arrays according to the present invention (Nolan JP, Sklar LA. (2002). Suspension array technology: evolution of the flat-array paradigm. Trends Biotechnol. 20(1):9-12). In such suspension arrays, the carrier, e.g. a microbead or microsphere, is present in suspension. The array consists of different microbeads or microspheres, possibly labeled, carrying different ligands.

Methods of producing such arrays, for example based on solid-phase chemistry and photolabile protective groups, are generally known (US 5,744,305). Such arrays can also be brought into contact with substances or substance libraries and tested for interaction, for example for binding or change of confirmation. Therefore, arrays comprising a peptide or polypeptide as defined above may be used for identifying ligands binding specifically to said peptides or polypeptides.

Peptides and polypeptides (proteins) can be measured in tissue, cell, and body fluid samples, i.e. preferably in vitro.

As mentioned above, the expression level of XCR1 is measured in a T cell sample. The term "T cell sample" relates to any kind of sample containing T cells in a manner suitable for measurement of the expression level of XCR1. Thus, the T cell sample may be a tissue sample or a body fluid sample. In particular, the T cell sample may be a blood sample.

According to the present invention, the term "tissue sample" relates to any kind of tissue containing T cells obtained from the dead or living body of a subject. Preferred tissue samples may be obtained from organs belonging to the immune system, such as thymus, lymph nodes, spleen and the like. Tissue samples can be obtained by any method known to the person skilled in the art, for example by biopsy or curettage.

According to the present invention, the term "body fluid sample" relates to any kind of body fluid containing T cells obtained from the dead or living body of a subject. Examples for body fluid samples include blood, blood serum, blood plasma, lymphe, cerebral liquor, saliva, vitreous humor, and urine. Samples of body fluids can be obtained by any method known in the art. Some of the samples, such as urine samples, may only contain degradation products, in particular fragments, of XCR1. However, as laid out above, measurement of the expression level of XCR1 may still be possible as long as the fragments are specific for XCR1.

It is well within the ability of the person skilled in the art to choose a sample suitable and advantagous in a particular setting. In many cases, a preferred sample according to the present invention is a blood sample. Even small blood samples contain a high number of T cells sufficient for reliable and representative measurement of XCR1. Blood samples are routinely obtained by minimally invasive procedures, e.g. during the regular check-up performed in transplant patients.

If necessary, the samples may be further processed before measurement. For example, T cells may be purified from other cell types (such as other blood cells) or tissue contained in the sample. Furthermore, nucleic acids, peptides or polypeptides may be purified from the sample according to methods known in the art, including filtration, centrifugation, or extraction methods such as chloroform/phenol extraction.

Furthermore, it is contemplated to use so called point-of-care or lab-on-a-chip devices for obtaining the sample and measuring XCR1. Such devices may be designed analogously to the devices used in blood glucose measurement. Thus, a patient will be able to obtain the sample and measure XCR1 without immediate assistance of a trained physician or nurse.

According to one embodiment, the measured expression level of XCR1 is compared with the expression level of XCR1 in a control T cell sample. Preferably, comparison is done via a reference value, wherein said reference value is representative for or derived from the expression level of XCR1 in a control T cell sample.

The terms "control" or "control T cell sample" are easily understood by the person skilled in the art. Preferably, the "control" relates to an experiment or test carried out to provide a standard, against which experimental results can be evaluated. In the present context, the standard preferably relates to the expression level of XCR1 associated with a particular tolerance status. Thus, a "control" is preferably a T cell sample taken to provide such a standard. E.g., the control T cell sample may be derived from one or more healthy subjects, or from one or more subjects representative of a particular tolerance status (or a particular disease condition or particular state of disease), as becomes also evident from the examples accompanying this specification. The control T cell sample may also have been derived from the same subject at an earlier time. Thus, the control T cell sample may be a standard representing the particular tolerance status or tolerance status in a particular condition or state of disease.

The person skilled in the art is familiar with the concept of reference values (or "normal values") for biochemical or molecular markers or other laboratory parameters. In particular, the term reference value may relate to the actual value of the expression level in one or more control T cell samples or it may relate to a value derived from the actual expression level in one or more control T cell samples. Preferably, T cell samples of at least 3, more preferably 10, more preferably 20, more preferably 50, most preferably 100 subjects are analyzed to determine the reference value.

In the most simple case, the reference value is the same as the expression level measured in the control sample or the average of the expression levels measured in a multitude of control samples. However, the reference value may also be calculated from more than one control sample. E.g., the reference value may be the arithmetic average of the expression level in control samples representing the control tolerance status (e.g. healthy, particular condition, or particular disease state). Preferably, the reference value relates to a range of values that can be found in a plurality of comparable control samples (control samples representing the same or similar tolerance status), e.g. the average ± one or more times the standard deviation. Similarly, the reference value may also be calculated by other statistical parameters or methods for example as a defined percentile of expression level found in a plurality of control samples, e.g. a 90 %, 95 %, or 99 % percentile. The choice of a particular reference value may be determined according to the desired sensitivity, specificity or statistical significance (in general, the higher the sensitivity, the lower the specificity and vice versa). Calculation may be done according to known statistical methods.

Thus, the term "representative" means that the reference value reflects the expression level of XCR1 in a control T cell sample.

If the measured expression level in the T cell sample to be analyzed is the same or similar to the reference value, it indicates that the T cell sample has the same or a similar tolerance status as the control sample.

If the measured expression level is higher or lower than the reference value, it indicates that the tolerance status deviates from the tolerance status of the control T cell sample.

According to a preferred embodiment, if the measured expression level is lower than the reference value, then it indicates that the T cell(s) of the sample are less tolerant than the T cells of the control T cell sample. In another preferred embodiment, if the measured expression level is higher than the reference value, then it indicates that the T cell(s) of the sample are more tolerant than the T cells of the control T cell sample.

Particularly, if the measured expression level of XCR1 is decreased (compared to the reference value or, more particularly, compared to the expression level in the control sample) by at least 2-fold, more preferably at least 3-fold, more preferably at least 5-fold, more preferably at least 10-fold, most preferably at least 20-fold, then it indicates that the T cell(s) of the sample are less tolerant than the T cells of the control T cell sample.

Particularly, if the measured expression level of XCR1 is increased (compared to the reference value or, more particularly, compared to the expression level in the control sample) by at least 2-fold, more preferably at least 3-fold, more preferably at least 5-fold, more preferably at least 10-fold, most preferably at least 20-fold, then it indicates that the T cell(s) of the sample are more tolerant than the T cells of the control T cell sample.

The latter two embodiments are based on the finding that a decrease in the expression level of XCR1 indicates a decreased tolerance status of the T cell(s) of the sample, whereas an increase in the expression level indicates an increase in the tolerance level of the T cell(s) of the sample. Analogously, the measured expression level in the T cell sample indicates the tolerance status of a subject or the immune system of subject from which the T cell sample was derived. The higher the increase or decrease in the expression level, the higher is the increase or decrease in the tolerance level.

The expression level of XCR1 of a particular T cell subpopulation in the T cell sample can be measured, wherein the reference value is representative of the expression level of XCR1 in a corresponding T cell subpopulation in the control sample.

According to this embodiment, it may not always be necessary to measure the expression level of XCR1 in the entire T cell sample. For example, the T cell sample may be purified from other immune cells, e.g. macrophages (which adhere to the bottom of a petri dish, and can thus be easily removed from the sample). Preferably, the expression level of XCR1 is measured only in the T cells contained in the T cell sample. It may be advantageous to measure the expression level of XCR1 in a particular T cell subpopulation and not in all T cells contained in the T cell sample. Purification of particular T cell subpopulations from a T cell sample can be done according to methods known by the person skilled in the art. Alternatively, the T cell subpopulation does not have to be purified from the T cell sample, but rather measurement may only be done in that particular subpopulation. For example, using FACS analysis, it is possible to measure different parameters on a particular cell in a given sample, which still contains other cells or impurities.

In another preferred embodiment, the particular T cell subpopulation is chosen from the group consisting of (i) cytotoxic T cells, (ii) helper T cells, (iii) regulatory T cells, (iv) CD8+ T cells, iv) CD8+ T cells, v) CD4+ T cells, vi) CD4+ CD25+ T cells, vii) CD4+ CD25- T cells, viii) a subpopulation having a T cell receptor subtype directed against a particular MHC or HLA subtype, ix) a subpopulation having a T-cell receptor subtype directed against a particular antigen.

Regulatory T cells in the context of the present invention are T cells with the capacity to control T cell homeostasis and limit or downmodulate any kind of immune responses during induction and/or effector/memory phase in an antigen-specific or antigen independent way.

The terms CD4+, CD8+, CD25+, CD 25-, and combinations thereof are known to the person skilled in the art. They relate to molecular or biochemical markers, which can be found on T cells. "+" indicates that the T cell expresses the marker, whereas "-" indicates that the T cell does not express the marker. The markers are being used to identify or characterize particular T cell subpopulations.

The term "T cell receptor" is also known to the skilled artisan. T cell receptors are antibody-like heterodimers which are present as membrane-bound molecules on the surface of T cells. A typical T cell receptor comprises two disulfide-linked polypeptide chains, the alpha- and a beta-chains, which together form the heterodimer (a small minority of T cells makes a different but related type of receptor heterodimer, composed of gamma-and delta-chains). The heterodimer can be noncovalently associated with a large set of other membrane-bound proteins.

Each polypeptide chain comprises a large extracellular part that is folded into two Ig-like domains: One variable (V) and one constant (C) domain. The V domains of alpha and beta chain form an antigen binding site, analogously to the antigen binding site of secreted antibodies. The antigen binding site of T cell receptors is generated by V(D)J recombination similar to the somatic recombination used in the generation of different antibodies. Analogously to B cells produding antibodies, each T cell or T cell clone produces only one subtype of T cell receptor with a given antigen binding site.

The terms "MHC" subtype and "HLA" subtype are known to the person skilled in the art. MHC proteins are called H-2 antigens (histocompatibility-2 antigens) in mice and and HLA antigens (human-leucocyte-associated antigens) in humans.

MHC (major histocompatibility complex) proteins are present on antigen-presenting cells (such as dendtritic cells). The MHC proteins bind peptide fragments of foreign antigens and present them to the T cells and thus can activate T cells whose T cell receptors recognize the peptide. The activated T cell (or effector T cell) can then recognize the same antigen on the surface of the target cell it influences (e.g. the destroys, in the case of a cytoxic T cell).

There are class I MHC proteins (e.g. HLA-A, HLA-B, and HLA-C), which present foreign peptides to cytotoxic T cells and class II MHC proteins (e.g. HLA-DP, HLA-DQ, and HLA-DR), which present foreign peptides to helper cells. Both class I (alpha chain and a beta2-microglobulin chain) and class II (alpha chain and beta chain) MHC proteins are heterodimers.

It should be noted that MHC proteins are the main antigens recognized in transplantat rejection reactions. One reason is that MHC proteins belong to the most polymorphic proteins in higher vertebrates: The number of alleles (alternative forms) of a single gene can reach more than 200. As each individual has at least 12 genes encoding MHC proteins, it is very rare for two unrelated individuals to have an identical set of MHC proteins. This makes it very difficult to match donor and receipient for organ transplantation. Furthermore, T cells are highly reactive against foreign MHC proteins: Whereas only 0.001% of T cells respond to a typical viral antigen, more than 0.1 % respond to a single foreign MHC antigen. Thus, a donor organ with only few MHC alleles can set off a massive T cell response.

In this context, the term MHC subtype relates to both different alleles of a given MHC gene or protein as well as different MHC gene proteins as such (e.g. HLA-A, HLA-B, HLA-C, HLA-DP alpha, HLA-DP beta, HLA-DQ alpha, HLA-DQ beta, HLA-DR alpha, HLA-DR beta).

By analyzing a T cell subpopulation having a T cell receptor subtype directed against a particular MHC or HLA subtype, it is possible to restrict analysis to T cells being directed against a transplant. This may be advantageous in the analysis or diagnosis of tolerance in the context of transplantation.

Furthermore, it may be advantageous to analyze a T cell subpopulation having a T cell receptor subtype directed against a particular antigen. An "antigen" is any substance capable of specific binding to a T cell receptor. In this context, the term antigen is to be understood in its broadest sense, e.g. the antigen can be an organ (e.g. a transplant), a cell (e.g. a transplanted cell, a tumor cell), a pathogen (e.g. a virus), or an allergen. The antigen can also be a particular molecule, e.g. a protein or peptide.

Preferably, the T cell subpopulation has a T cell receptor subtype directed against a particular epitope of said antigen. The term "epitope" is known by the person skilled in the art. In the context of the present invention, an epitope (also known as antigenic determinant) is a particular site on an antigen molecule which binds to a specific T cell receptor and/or activates a specific T cell subpopulation carrying such specific T cell receptor.

Analysis or identification of the T cell subpopulation can be carried out by providing the antigen or the epitope and measuring the binding of the T cell or the T cell receptor.

In another embodiment, the present invention relates to the use of the expression level of XCR1 for measuring the tolerance status of a T cell sample.

In another embodiment, the present invention relates to a method for diagnosing or monitoring of an immune response, comprising the steps of
a) measuring the expression level of XCR1 in a T cell sample,
b) comparing the measured expression level with a reference value, said reference value being representative of the expression level of XCR1 in a known state of the immune response.

Particularly,
c) if the measured expression level is lower than the reference value, then it indicates that the immune response is stronger compared to the known state, or
d) if the measured expression level is higher than the reference value, then it indicates that the immune response is weaker compared to the known state.

Furthermore, in another embodiment, the present invention relates to the use of the expression level of XCR1 for diagnosing or monitoring of an immune response.

The terms "diagnosing" and "monitoring" are known to the person skilled in the art. In particular, the term "diagnosing" according to the present invention relates to confirming, subclassifying or predicting the relevant immune response, tolerance status, disease, or condition. Confirming is understood as strengthening or substantiating a diagnosis already performed using other indicators or markers. Subclassifying relates to further defining a diagnosis, e.g. according to mild or severe forms of the disease. Predicting relates to prognosing a disease, in particular before other symptoms or markers have become evident or have become significantly altered. Monitoring relates to keeping track of an already diagnosed immune response, tolerance status, disease, or condition, e.g. to analyze the progression of a disease or the influence of a particular treatment.

The term immune response is known to the person skilled in the art. During an immune response specific antibodies and/or cytotoxic T cells are produced. Preferably, the term immune response in the present context relates to a cell-mediated or cellular immune response, i.e. an immune response mediated by T cells. Such an immune response is typically characterized by the activation or clonal expansion of T cells (clonal expansion relates to the proliferation of particular T cell clones). Preferably, the term immune response relates to an immune response of a subject, and the T cell sample is derived from the subject.

A weaker immune response results in reduced production of antibodies, or, particularly, cytotoxic T cells. A stronger immune response results in increased production of antibodies, or, particularly, cytotoxic T cells.

In particular, the term immune response relates to a selective response mounted by the immune system of a subject against a transplant, a self-antigen, a pathogen, or an allergen.

The term "transplant" is known to the person skilled in the art. In the context of the present invention, the term transplant relates to any cell, tissue, or organ being transferred (transplanted or "grafted") from the body of one subject into the body of another subject. According to the present invention, the term transplant does not relate to a cell, tissue, or organ transferred from the one part of the body to another part of the body of the same individual. Neither does the term transplant relate to a cell, tissue, or organ being explanted and then re-implanted into the same individual (except if the re-implanted cell or tissue has been antigenically altered, e.g. by genetic engineering).

The transplant can be grafted into a genetically identical (e.g. twins) or genetically non-identical subject. The teaching of the present invention is particularly advantageous in the case of a transplant grafted into an genetically non-identical subject. Such a transplant can be an allotransplant (if the subjects belong to the same species) or an xenotransplant (if the subjects belong to different species). Donors for xenotransplants include non-human primates or pigs. The donor may be dead or alive. Preferably, the transplant is of approximately the same size as the organ of the recipient. However, also smaller transplants may be grafted and may be allowed to grow to their appropriate size in situ (e.g. split livers).

Furthermore, the teaching of the present invention is particularly advantageous in the case of transplantation into immunologically non-privileged parts of the body (particularly those parts of the body not belonging to the central nervous system).

The transplant can be any cell or tissue grafted by the person skilled in the art for the treatment of cell, tissue, or organ failure or malfunction, particularly in the case of bone marrow, kidney, liver, heart, lung, pancreas. The transplant can also be grafted in the context of treating diabetes, liver cirrhosis, heart disease, Parkinson's disease, Alzheimer's disease, AIDS, cancer, or any complications associated with such diseases or associated with treatment of such diseases (e.g. chemotherapy).

For example, the transplant may be a cell, e.g. chosen from the group consisting of blood cells, bone marrow cells, adult stem cells (e.g. mesenchymal stem cells), heart cells (e.g cardiomyocytes), nerve cells (e.g. dopamine secreting cells), liver cells (e.g. hepatic progenitor cells), and pancreatic cells (e.g. pancreatic islet cells, more particularly alpha cells or beta cells). The cell may be genetically modified or otherwise pretreated. In particular, a stem cell may have been differentiated according to procedures known in the art to produce differentiated cells. Preferably, a plurality of cells is transplanted, more preferably at least 10, more preferably at least 100, more preferably at least 1000, more preferably at least 10⁴, most preferred at least 10⁵ cells are transplanted. The transplant may also be tissue, e.g. chosen from the group consisting of skin tissue, liver tissue, heart tissue (particularly heart muscle), and pancreatic tissue. The tissue may be grafted from a natural donor or it may originate from tissue culture. The transplant may also be an organ or part of an organ, e.g. kidney, liver, split liver, heart, heart valve, lung, pancreas, duodenum, colon, cornea, meninges, ossicle, or blood vessel.

A "self-antigen" (or autoantigen) is any cell, cell part, or molecule of a subject's own body which is recognized by the subject's own immune system, particularly eliciting an autoimmune response. Although whether or not a certain substance acts as a self-antigen depends on the particular subject and substance, there are many examples for substances which frequently act as self-antigens. Such self-antigens and/or autoantibodies directed against the respective self-antigens are well known to the person skilled in the art. Examples for such self-antigens include parietal cells, smooth muscle (SMA, actin), liver or kidney microsomes, heart muscle, skeletal muscle, filaggrin, fibrin, keratin, citrullinated proteins or peptides, thyroid antigens, neuronal nuclear antigens, hypophysis, adrenal cortex, cell nuclei (e.g. DNA or histones), mitochondria, actin fibers, reticular fibers (ARA), ribosomal proteins, centrioles, cyclins, pancreatic beta-cells.

The term "pathogen" relates to any disease-causing virus or microorganism. As the teaching of the present invention is particularly advantageous in the context of a pathogen eliciting a cellular immune response. Such a pathogen typically spends part of its life cycle within a cell of the subject, e.g. it is a virus (e.g. a retrovirus) or an intracellular microorganism (e.g. Mycobacterium tuberculosis).

An "allergen" is a substance to which an individual subject is hypersensitive and which causes an immune response in the subject, often characterized by local inflammatory reactions but sometimes by severe shock symptoms (anaphylactic shock). By their mode of uptake or generation, allergens are characterized as contact allergens, food allergens, ingestant allergens, inhalant allergens, or photoallergens. Although whether or not a certain substance acts as an allergen depends on the particular subject, there are many examples for substances which frequently act as allergens. Such examples include pollen, fungal conidia, animal epithelia (e.g. animal hair), mite feces, penicillin, formaldehyde, flour, and milk protein.

An allergy can be primarily humoral or cell-mediated. The present teaching is particularly advantageous in the case of cell-mediated (or cytotoxic) allergies which is not part of the present invention. In another embodiment, the present invention relates to a method for diagnosing or monitoring of a transplant rejection reaction or graft-versus-host disease, comprising the steps of
a) measuring the expression level of XCR1 in the T cell sample,
b) comparing the measured expression level with a reference value, said reference value being representative of the expression level of XCR1 in a known state of the disease,
c) if the measured expression level is lower than the reference value, then it indicates that the disease is more severe compared to the known state, or
d) if the measured expression level is higher than the reference value, then it indicates that the disease is less severe compared to the known state.

Furthermore, in another embodiment, the present invention relates to the use of the expression level of XCR1 for diagnosing or monitoring of a transplant rejection reaction or graft-versus-host disease.

The term "transplant rejection reaction" relates to an undesired immune response directed against a transplant. Eventually, such an immune response may damage or completely destroy the transplant. The transplant rejection reaction may be acute or chronic. The severity of a transplant rejection may vary, for example, according to the type of transplant (e.g. liver is usally better accepted by the recipient than kidney or bone marrow) and the degree of match between the MHC antigens fo donor and reciepient.

The term "graft-versus-host-disease" relates to an immune reaction mounted by immunocompetent cells of the graft attack cells or tissue of the host, particularly in a cell-mediated immune response. Graft-versus-host-disease is frequently observed after inadvertent or intended transplantation of immunocompetent cells from bone marrow, lymph nodes or spleen. Graft-versus-host-disease is particularly observed in subjects treated with immunosuppressants (in healthy subjects, the transplanted immunocompetent cells are usually detected and destroyed by the subject's immune system). A special form of graft-versus-host-disease is Runt disease, which may occur, for example, if maternal lymphocytes transfer into the developing fetus.

The term "autoimmune disease" relates to a large group of diseases in which the body mounts an immune response against its own components. Such immune response may lead, for example, to chronic inflammation or tissue destruction.

Autoimmune diseases can be organ-specific (the immune reaction is directed exclusively against specific antigens of a particular organ or organ system), non-organ-specific (particularly diseases of the so-called rheumatic group of diseases), or they can be a hybrid or crossover between organ-specific and non-organ-specific forms.

Examples for organ-specific autoimmune diseases include diseases affecting the thymus (e.g. Hashimoto-Thyreoditis), stomach (pernicious anemia, chronic gastritis), pancreas (e.g. juvenile diabetes mellitus), or adrenal gland (Addison's disease).

Examples for non-organ-specific autoimmune diseases include diseases with immune responses against autoantigens of different body parts, possibly with deposits of immune complexes, primarily of joints (e.g. rheumatic arthritis), kidney (e.g. systemic Lupus erythematodes), skin (scleroderma), or muscle (dermatomyositis).

Examples for hybrid or crossover forms include Goodpasture's syndrome, myasthenia gravis pseudoparalytica, pemphigus vulgaris, bullous pemphigoid, ophtalmia sympathica, phacogenic uveitis, autoimmune hemolytic anemia, Werlhof's disease, primary biliary cirrhosis, chronic aggressive hepatitis, colitis ulcerosa, Sjögren's syndrome. It is likely that also multiple sclerosis belongs to this group of autoimmune diseases.

Allergic reactions or allergies are hypersensitive immune reactions exhibited by certain subjects or individuals on exposure to an otherwise innocuous antigen (e.g. pollen, food, drugs, dust-mites, etc.). Some allergies (e.g. hay-fever) involve the production of IgE antibodies which cause the release of inflammatory products such as histamine from mast cells, causing the characteristic symptoms. Other allergies, particularly some contact-mediated sensitivities, are caused by T cell mediated reactions. The present teaching is particularly advantageous in the case of T cell mediated allergic reactions or aspects of allergic reactions mediated by T cells, but is not within the scope of the present invention.

Immunosuppressants are known to the person skilled in the art. Immunosuppressants are any drugs, agents or treatment regimens capable of suppressing the ability to mount a normal immune response. Typically, the immunosuppression caused by the immunosuppressant is only of temporary nature. Immunosuppressant drugs may be administered systemically or locally (e.g. in atopic dermatitis).

Immunosuppressants may include agents capable of
a) binding to cyclophilins or FKBP-12,
b) binding to TOR (target of *r*apamycine) proteins
c) inhibiting IL-2 synthesis or IL-2 signal transduction,
d) inhibiting T cell proliferation,
e) inducing cell death in T cells,
f) inhibiting or blocking co-stimulation of T cells, or
g) any combination of a) to f).

Examples for immunosuppressants include cyclosporines (e.g. cyclosporin A), Tacrolismus (FK506), Sirolismus (rapamycine), and antimetabolites (e.g. azathioprines, mycophenolate mofetil),

Further examples for immunosuppressants include immunosuppressant antibodies or derivatives thereof (e.g. humanized antibodies, chimeric antibodies, or antibody fragments). Particular examples are antibodies directed against interleukin-2 receptor (CD25) (e.g. Basiliximab, Daclizumab), lymphocytes (ATG), CD3 (e.g. OKT3 (an antibody of murine origin directed against human CD3ε)), CTLA4, or CD4. Possibly also antibodies directed against CD52 may be used. ATG and OKT3 cause cell death of T cells. anti-CTLA4 inhibits co-stimulation.

The antibodies may also be conjugated, covalently or non-covalently, with toxins (e.g. an antibody against CD25 may be coupled to the A chain of ricin toxin).

Immunosuppressant treatment regimens may include irradiation and/or killing of lymphocytes.

As laid out above, so-called anti-inflammatory drugs or agents may also be used as immunosuppressants. Examples include steroids (particularly corticosteroids or glucocorticoids) as well as non-steroid anti-inflammatory drugs (NSAIDs). Examples for anti-inflammatory drugs include cortisone, fluocortolone, hydrocortisone, prednisolone, methylprednisolone, prednisone, prednylidene, and Cox-2 inhibitors (e.g. Celecoxib or rofecoxib (VIOXX)).

Particularly, diseases treatable or treated with immunosuppressants include transplant rejection reactions, graft-versus-host-disease, autoimmune disease, and allergic reactions. These diseases are know to the person skilled in the art and will be described in more detail further below in this document.

"Known state of the disease" refers to a known degree of severity or progression of the disease. In particular, the known state of the disease concerns any aspect of the disease influenced by the immune system, particularly by the tolerance status of the immune system or its T cells. The known state may, for example, be determined from a pool of subjects or patients suffering from the same disease or it may be defined by the state of the disease observed in the same patient at an earlier time. A suitable reference value can then be determined analogously to the methods laid out earlier in this specification.

An in vitro method for determining the required stringency of immunosuppression in a subject suffering from a transplant rejection reaction or graft-versus-host disease who is already treated by immunosuppression, comprising the steps of
a) measuring the expression level of XCR1 in a T cell sample of a subject,
b) comparing the measured expression level to a reference value, said reference value being representative of the expression level of XCR1 in a T cell sample from a control subject requiring a known stringency of immunosuppression,
c) if the measured expression level is lower than the reference value, then it indicates that the stringency of immunosuppression should be maintained at the current level or increased compared to stringency of immunosuppression required by the control subject, or
d) if the measured expression level is higher than the reference value, then it indicates that the stringency of immunosuppression may be lowered compared to stringency of immunosuppression required by the control subject.

Advantageously, the present invention allows to determine the required stringency of immunosuppression in a subject or patient. The term immunosuppression relates to any methods or means (e.g. immunosuppressants) suppressing the ability of the immune system to mount a normal immune response. Typically, the immunosuppression is only of temporary nature and can be adapted, e.g. by changing the dosage of an immunosuppressant administered to a subject or patient. The required stringency relates to the minimal level of immunosuppression necessary to suppress an undesired immune response, as already explained further above.

Therefore, the term "determining the required stringency of immunouppression" particularly relates to the "determining the required dosage of an immunosuppressant" to be administered to a subject or patient. Thus, the present invention allows to determine the required dosage of an immunosuppressant to be administered to a subject or patient.

As laid out earlier, the required stringency of immunosuppression or the required dosage of a immunosuppressant depends on the tolerance status of the immune system, particularly the T cells, of the subject. As the present invention allows to determine the tolerance status, it also allows to determine the required stringency of immunosuppression.

This and similar embodiments help to solve a long-standing problem, namely to adjust or optimize the dosage of an immunosuppressant to be administered to a subject. In particular, these aspects of the invention allow to reduce the stringency of immunosuppression, in particular the dosage of immunosuppressant, to the necessary minimum. Thus, undesired side-effects from immunosuppression, particularly immunosuppressants, can be reduced. It should also be kept in mind that strong immunosuppression makes a subject susceptible to infections by pathogens. Reducing immunosuppression will allow the subject to better defend itself against pathogens.

The tolerance status can differ strongly from subject to subject and even within the same subject over time. Thus, the minimal required dosage depends strongly on the individual subject and/or, e.g., the individual transplant the subject has received. Therefore, the chosen level of immunosuppression or dosage of an immunosuppressant is frequently considerably higher than necessary (for example to ensure that no rejection or graft-versus-host disease occurs) and may cause severe undesired side-effects.

The present invention allows to lower the stringency or dosage to a minimal safe level. For example, if a control subject with the same or a similar expression level of XCR1 in a T cell sample requires a certain stringency or dosage of immunosuppressant, then the dosage of immunosuppressant administered may be determined to be of the same or a similar level.

Again, the person skilled in the art will be able to determine a suitable reference value according to the methods and considerations described out earlier in this specification. In a preferred method, the reference value is determined from a statistical analysis of the expression level of XCR1 in comparison to the stringency or dosage of immunosuppressant administered and the outcome concerning undesired immune responses and side-effects. Methods to carry out such statistical analyses, prospective or retrospective, are well known to the person skilled in the art.

The term "required dosage" relates to the minimal level of immunosuppressant necessary to suppress an undesired immune response. The undesired immune response is a transplant rejection reaction for a graft-versus-host-disease.

It will be appreciated that the expression level of XCR1 serves as guidance in determining the required dosage and that more factors may influence the determining a particular dosage administered. Such other factors may include the rate of drug clearance (e.g. through liver or kidney function), the general health status, the weight, the age, or the sex of the subject or patient. In transplant patients, the transplant function or any damage already caused by side-effects of the immunosuppressant(s) may be taken into consideration. Therefore, a final decision, e.g. about the actual dosage of an immunosuppressant to be administered, will be made by a physician.

It will also be appreciated that treatment with immunosuppressants frequently involves administering one or more different immunosuppressants. The person skilled in the art is able to determine a total dosage or a suitable combination of dosages for each immunosuppressant administered. Thus, the term "dosage" may relate to the combined dosages of each immunosuppressant.

"Lowering" the dosage may also relate to switching treatment to a less potent or better tolerated immunosuppressant. Analogously "increasing" the dosage relates to switching treatment to a more potent or powerful immunosuppressant, possibly at the cost of increased side-effects. The relative potency and severity of side-effects of different immunosuppressants are known to the person skilled in the art.

In another embodiment, the present invention relates to an vitro method for determining the required stringency of immunosuppression in a subject suffering from a transplant rejection reaction or graft-versus-host disease who is already treated by immunosuppression, comprising the steps of
a) measuring the expression level of XCR1 in a T cell sample of the subject,
b) comparing the measured expression level to the expression level of XCR1 in a control T cell sample obtained from the subject at an earlier time,
c) if the measured expression level is lower than the expression level in the control T cell sample, then it indicates that the stringency of immunosuppression should be maintained at the current level or increased compared to the stringency at the earlier time, or
d) if the measured expression level is higher than the expression level in the control T cell sample, then it indicates that the stringency of immunosuppression may be lowered compared to the stringency the earlier time.

Most subjects or patients treated by immunosuppression are regularly seen for monitoring of the disease progression or success of the treatment. E.g. transplant patients typically receive regular monitoring every four weeks. According to the above method, it is contemplated to use the expression level of XCR1 to follow the development of tolerance and consequently as a guidance for regular optimization of the required dose of immunosuppressant. In this context, it is important to note that tolerance may not always be permanent and that monitoring the tolerance status is important to early detect a loss in tolerance, which may require to increase the stringency of immunosuppression.

It should be understood that there are treatment regimens currently considered or applied in the art to actively induce immunological tolerance. Examples for such treatment regimens include administration of antibodies, particularly monoclonal antibodies, directed against cell surface molecules of T cells such as anti-CD3, anti-CD4, or anti-CD40 ligand (e.g. Winsor-Hines, D., Merrill, C., et al. (2004). Induction of Immunological Tolerance/Hyporesponsiveness in Baboons with a non-depleting CD 4 antibody. The Journal of Immunology, vol. 173, pp. 4715-4723). Another example is the administration of a weak agonistic antigen (see e.g. Strom, T. B. (2004). Is transplantation tolerable? The Journal of Clinical Investigation, vol. 113, pp. 1681-1683).

The methods and uses described herein are expressly also considered for evaluating the success (i.e. the development of tolerance) of such treatment regimens. Such evaluation may concern the treatment regimen as such (e.g. through statistical analyses) as well as the success in an individual patient.

In another embodiment, the present invention relates to the use of the primers defined by 5'-GTC-CAG-ACA-GAG-ACA-CCG-AAC-A-3' and 5'-GCC-CAG-CTG-AGG-AAG-TAT-GC-3' for measuring the expression level of XCR1 in a T cell sample of a subject suffering from transplant rejection reaction or graft-versus-host disease, for determining the required stringency of immunosuppression in a subject suffering from a disease treatable or already treated by immunosuppression.

Means suitable for measuring the expression level of XCR1, such as antibodies, aptamers, antisense nucleic acids etc. have been already been described in detail earlier in this specification. The means is packed as a kit comprising a means suitable for measuring the expression level of XCR1 and, preferably, an auxiliary agent for measurement. Preferably, the kit comprises a container for the means or agent for measurement and, preferably, a container for at least one auxiliary agent for measurement, e.g. a suitable buffer, filter, column, enyzme, etc.. The auxiliary agent may also be a means required for preparation of the sample, e.g. for obtaining or purifying of the sample, e.g. a blood sample. The auxiliary agent may also be an agent suitable for identifying T cells or a subpopulation of T cells. Suitable agents have been described ealier in this specification. The means can also be a lab-on-a-chip device or any other device suitable for point-of-care diagnosis. Devices for point-of-care diagnosis are known in the art. Preferably, such a device comprises a sampling unit (e.g. for taking a blood sample) and a measurement unit (e.g. for measuring the binding of XCR1 to a ligand). The means may also be a test strip or other auxiliary tool for measuring the expression level of XCR1 in such a device.

Therefore, the present invention also describes to a kit, a lab-on-chip device, or a point-of-care diagnostic device for measuring the expression level of XCR1.

In another embodiment, the present invention relates to an in vitro method to identify or validate an inhibitor or inducer of T cell tolerance in transplant rejection reactions or graft-versus-host disease, comprising the steps of
a) providing a T cell,
b) bringing the T cell in contact with a candidate for an inhibitor or inducer,
c) measuring the expression level of XCR1 in the T cell of step b), and
d) comparing the measured expression level with the expression level of XCR1 in the T cell before step b), or
e) comparing the expression level with expression level of XCR1 in a corresponding T cell not brought in contact with the candidate for an inhibitor or inducer.

As the teaching of the present invention allows to measure the tolerance status of a T cell or an immune system, it allows to identity substances that inhibit or induce immunological tolerance or the tolerance status of a T cell. The person skilled in the art is aware of a great variety of suitable means and methods in such identification or screening methods.

In particular, suitable methods may include in vitro assays (in particular cell culture systems) or animal models.

In a preferred embodiment, the method may comprise additional step (a1) measuring, the expression level of XCR1 in the T cell, said step (a1) being carried out before step (b).

In another embodiment, the present invention relates to a method to identify or validate an inhibitor or inducer of T cell tolerance or immune system tolerance in transplant rejection reactions or graft-versus-host disease, comprising the steps of
(a) providing a non-human animal,
(b) exposing the subject to a candidate for an inhibitor or inducer,
(c) measuring, preferably in vitro, the expression level of XCR1 in a T cell sample obtained from the subject after step b), and
(d) comparing the measured expression level with the expression level of XCR1 in a T cell sample obtained from the subject before step (b), or
(e) comparing the measured expression level with the expression level of XCR1 in a corresponding T cell sample obtained from a corresponding subject not brought in contact with the candidate for an inhibitor or inducer,
   wherein methods of treatment of the animal body are to be excluded. In a preferred embodiment, the method may comprise additional step (a1) measuring, preferably in vitro, the expression level of XCR1 in a T cell sample obtained from the subject, said step (a1) being carried out before step (b).

The candidate for an inhibitor or candidate for an inducer may be any substance deemed appropriate, e.g. chosen from the group of nucleic acids, antisense nucleic acids, peptide nucleic acids, siRNAs (short interfering RNAs), proteins, peptides, cytokines or derivatives thereof, antibodies, aptamers, inorganic compounds, organic compounds, and low molecular weight molecules (LMWs). The candidate may be an artificial or a naturally occuring substance. The candidate may also be chosen in a random or unbiased way, e.g. from a library of compounds.

However, the candidate may be also designed according to assumptions about possible biochemical pathways leading to immunological tolerance or targets capable of mediatin immunological tolerance. The candidate may also have been chosen according to any other consideration (e.g. similarity to known compounds inducing tolerance, e.g. particular antibodies such as mentioned above). Such a candidate is more likely to be an inducer or inhibitor than a randomly chosen canditate. In this case, the method according to the present invention rather relates to validation than identifcation of the inducer or inhibitor.

Particularly suited for validation is any in vivo method, wherein a non-human animal is exposed to the candidate. In non-human animals, the degree of validation will be better the more similar (phylogenetically) the animal is to the human.

LMWs molecules are molecules which are not proteins, peptides antibodies or nucleic acids, and which exhibit a molecular weight of less than 5000 Da, preferably less than 2000 Da, more preferably less than 2000 Da, most preferably less than 500 Da. Such small molecules may be identified in high throughput procedures/screening assays starting from libraries. Such methods are known in the art. Suitable small molecules can also be designed or further modified by methods known as combinatorial chemistry.

Furthermore, the present invention describes to the use of the expression level of XCR1 to identify an inhibitor or inducer of tolerance of a T cell or of the immune system of a subject.

Analogous methods may be designed to identify or validate treatment methods which influence the tolerance of a T cell or of the immune system of a subject. Instead of the candidate for the inhibitor or inducer, a candidate for a treatment method is used. Preferably, the candidate for a treatment method is a method expected to increase the tolerance of the T cell or the immune system.

Consequently, the present invention also describes to the use of the expression level of XCR1 to identify or validate a treatment method which influences the tolerance of a T cell or of the immune system of a subject.

All identification or validation methods may be adapted for high-throughput purposes, e.g. several samples may be processed and analyzed in parallel, e.g. in a 24-well, 96-well, 356-well, or 1024-well format.

Similarly, said inhibitor or activator may be provided by any of the screening methods described above and formulated into a pharmaceutical composition.

The term "adequate amounts" is understood by the person skilled in the art. Preferably, adequate amounts relate to pharmaceutically effective amounts. As used herein, a "pharmaceutically effective amount" of an inducer or inhibitor is an amount effective to achieve the desired physiological result, either in cells treated in *vitro* or in a subject treated *in* vivo. Specifically, a pharmaceutically effective amount is an amount sufficient to influence, for some period of time, T cell tolerance, in particular to increase T cell tolerance. The pharmaceutically effective amount may vary depending on the specific inducer or inhibitor selected, and is also dependent on a variety of factors and conditions related to the subject to be treated and the severity of the disease. For example, if the inducer or inhibitor is to be administered *in vivo,* factors such as age, weight, sex, and general health of the patient as well as dose response curves and toxicity data obtained in pre-clinical animal tests would be among the factors to be considered. If the inducer or inhibitor is to be contacted with cells *in vitro,* one would also design a variety of pre-clinical *in vitro* studies to asses parameters like uptake, half-life, dose, toxicity etc. The determination of a pharmaceutically effective amount for a given agent (inducer or inhibitor) is well within the ability of those skilled in the art. Preferably, the inducer or inhibitor is present in a concentration of 0,1 to 50% per weight of the pharmaceutical composition, more preferably 10 to 30%. The pharmaceutical composition may comprise a pharmaceutically acceptable carrier and/or substances able to influence bioavailability and/or stability of the inducer or inhibitor.

### Figure legends

Fig. 1:
   Sequence alignment of the protein sequences of mouse, human, pig, and rat XCR1 (Genebank designation numbers NM_011798, AY275469, NM_001001622, XP_236740) were compared using the DNASTAR MegAlign software (GATC Biotech AG, Konstanz, Germany; Martinez/Needleman & Lipman-Pearson method). M, mouse; h, human; p, pig; r, rat; maj, majority.

### Example 1

KeratinIV.Kb and Des.TCR single-transgenic mice and double-transgenic mice were crossed to the RAG-2 deficient background (Alferink J, Tafuri A, Vestweber D, Hallmann R, Hammerling GJ, Arnold B. Control of neonatal tolerance to tissue antigens by peripheral T cell trafficking. Science 1998: 282(5392); 1338-41). Wild type mice were obtained from Charles River Laboratories. Mice used for experiments were 6-12 weeks old. All mice were kept under specific pathogen-free conditions at the central animal facility of the German Cancer Research Center.

### Transfected cell lines

The murine mastocytoma cell line P815 was transfected with the genes encoding for the murine MHC class I molecule H-2Kb and the B7-1 gene plus the neomycine resistance gene using the calcium phosphate method (Limmer A, Sacher T, Alferink J, Kretschmar M, Schönrich G, Nichterlein T, Arnold B, Hämmerling GJ. Failure to induce organ-specific autoimmunity by breaking of tolerance: importance of the microenvironment. Eur. J. Immunol. 1998: 28; 2395-2406). Transfected cell lines were cultured in Dulbecco's modified Eagle's medium (DMEM) containing 10% FCS, 10 mM hepes, 200 mM L-glutamine, 100 mM sodium pyruvate and 10 000 E/10 mg/ml penicilline/strepto-mycine. For s.c. injection the cells were trypsinized (2 ml 0.25% trypsin plus 2.5 mM EDTA for 5 min at room temperature) and washed with Dulbecco's PBS (DPBS).

### Cell isolation

Single-cell suspensions of spleens were prepared from mice of the indicated strains. Total CD8⁺ T cells were purified using CD8⁺-specific microbeads (Miltenyi Biotec, Bergisch Gladbach, Germany) according to the manufacturers instructions.

### Antibodies and flow cytometry

The CD4, CD8 and CD25 monoclonal antibodies were obtained from BD Biosciences. Flow cytometric analyses were done on a FACSCalibur with the CellQuest software (BD Biosciences). To obtain highest purities (99,6% and higher) MACS isolated cells were sorted on a FACSVantage (BD Biosciences).

### Real-time RT-PCR

RNA was isolated with the RNeasy kit (Qiagen, Hilden, Germany) following cDNA synthesis using the Superscript II Reverse Transcriptase (Invitrogen). Quantitative real-time RT-PCR was performed in a Gene Amp 5700 Sequence Detection System (Applied Biosystems) using a SYBR Green PCR kit from Applied Biosystems. Each sample was assayed in triplicates and a 25µl final reaction volume was used. Primer pairs were designed using Primer Express software and synthesized by Applied Biosystems. A threshold was set in the linear part of the amplification curve, and the number of cycles needed to reach it was calculated for every gene. Melting curves established the purity of the amplified band. Primer sequences are: mXCR1 FP 5'-GTC-CAG-ACA-GAG-ACA-CCG-AAC-A-3', RP 5'-GCC-CAG-CTG-AGG-AAG-TAT-GC-3'; HPRT FP 5'-ACA-CCT-GCT-AAT-TTT-ACT-GGC-AAC-A-3'; RP 5'-TGG-AAA-AGC-CAA-ATA-CAA-AGC-CTA-3'. The relative mRNA frequencies were determined in relation to the house keeping gene HPRT. Briefly, we calculated for each sample the difference in the threshold cycles (CT) between the target gene and HPRT: ΔCT sample = (CTₛₐₘₚₗₑ - CT_{HPRT}). Relative mRNA frequencies were calculated as 2^{ΔCT} and the experimental samples were normalized to the respective calibrator.

### DNA microarray and data analysis

Total RNA of mouse T cells was isolated using RNeasy kits with DNase I digestion on column (Qiagen, Hilden, Germany). RNA quality was checked using RNA 6000 Nano Assays performed on the Bioanalyzer 2100 Lab-on-a-Chip system (Agilent Technologies, Palo Alto, CA, USA). The following modifications to the protocol of Baugh et al. (Baugh LR, Hill AA, Brown EL, Hunter CP. Quantitative analysis of mRNA amplification by in vitro transcription. Nucleic Acids Res. 2001: 29(5):E29, and http://www.mcb.harvard.edu/hunter) were performed: 200 ng RNA was combined with 100 ng oligo(dT)-T7 primers in a total RT volume of 10 µl. T4gp32 (USB, Cleveland, OH, USA) was used at final amounts of 0,8 mg/ml. The reaction was incubated for 1 h at 50 °C. Round 1 cRNA was purified according to the RNA-cleanup protocol (RNeasy Mini kit, Qiagen) and coprecipitated with LPA (Ambion, Huntingdon, UK). Round 2 amplification (cRNA labeling) was performed according to Affymetrix instructions (see e.g. Kenzelmann M, Klaren R, Hergenhahn M, Bonrouhi M, Grone HJ, Schmid W, Schutz G. High-accuracy amplification of nanogram total RNA amounts for gene profiling. Genomics. 20004: 83(4); 550-8).

Amplified cRNAs were profiled on Affymetrix murine U74Av2 (Affymetrix, Inc., Santa Clara, CA, USA) according to the manufacturer's instructions. After hybridization the Gene Chips were washed, stained with SA-PE and read using an Affymetrix GeneChip fluidic station and scanner. Data were analyzed using the MAS 5.0 version of the Affymetrix software packages. For normalization, all array experiments were scaled to a target intensity of 150, otherwise using the default values of the Microarray suite. Genes are considered as differentially regulated when their fold change is greater than or equal to 2, or less than or equal to -2, the statistical parameter for a significant change is less than 0.01 [change p-value for changes called increased (I)] or greater than 0.99 [change p-value for changes called decreased (D)].

### Example 2

The methods in this example were carried out as described in Example 1.

### Des.TCRxKeratinIV.Kb mice are tolerant in the absence of CD4 cells

Previous work had shown that selective expression of the MHC class I molecule Kb in the skin of transgenic mice results in non-deletional tolerance as verified by the use of respective T cell receptor transgenic mice (Des.TCR) (Alferink J, Tafuri A, Vestweber D, Hallmann R, Hammerling GJ, Arnold B. Control of neonatal tolerance to tissue antigens by peripheral T cell trafficking. Science 1998: 282(5392); 1338-41). Tolerance is induced in the neonatal stage when skin is accessible for naïve T cells and maintained by regulatory Des.TCR CD8+ T cells during adulthood when naive cells have restricted access to healthy tissue. These mice strains were crossed to the RAG 2 -/- background to obtain a T cells population devoid of other than the Kb-specific CD8 T cells.

The DES.TCRxKeratinIV.Kb thymectomized mice were the source to purify the tolerant CD8+ T cells from. The mice had been thymektomized to enrich the tolerant CD8+ T cells which did have contact with the Kb molecule expressed in the skin. Tolerant mice did accept the antigen specific tumor graft P815KbB7. Control mice were DES.TCR mice that had rejected the tumor P815KbB7 and were also thymektomized for control reasons.

### Array analysis identifies mXCR1 as upregulated in tolerant DES.TCR CD8 cells

For the identification of markers of tolerant CD8+ cells we compared the gene expression profiles of tolerant and activated T cells. CD8+ T cells were purified using the MACS technology and further enriched via sorting (FACS) to obtain up to 99,9% pure cell populations. RNA was isolated from the purified T cells and after linear amplification analyzed by microarrays (Affymetrix murine U74Av2 arrays). Within the upregulated genes of the tolerant CD8+ T cells we identified the murine lymphotactin receptor (mXCR1, see Yoshida T, Izawa D, Nakayama T, Nakahara K, Kakizaki M, Imai T, Suzuki R, Miyasaka M, Yoshie O. Molecular cloning of mXCR1, the murine SCM-1/lymphotactin receptor. FEBS Lett. 1999: 458(1); 37-40) (Table 1).

### RT-PCR confirms the upregulation of mXCR1

The upregulation of mXCR1 could be verified by using RT-PCR as a different technical approach (Table 1 and 2). Separate samples of RNA were prepared to obtain these results. Samples of naïve DES.TCR CD8+ T cells showed also no upregulation of mXCR1.

**Table 1: Expression data for mXCR1 in CD8+ T cells. The average and standard deviation fold change values for the respective experiment are depicted. The results were obtained from two (microarrays) and four (RT-PCR) independent experiments.**

| Gene | Array data Avg. Fold Change tolerant vs. activated | Array data Std. Fold Change tolerant vs. activated | RT-PCR data Avg. Fold Change tolerant vs. activated | RT-PCR data Std. Fold Change tolerant vs. activated |
|---|---|---|---|---|
| mXCR1 | 2.7 | 0.2 | 11.9 | 7.4 |

**Table 2: Expression data for mXCR1 in CD8+ T cells. The average and standard deviation fold change values for the respective experiment are depicted. The results were obtained from four independent experiments.**

| Gene | RT-PCR data Avg. Fold Change tolerant vs. naive | RT-PCR data Std. Fold Change tolerant vs. naive |
|---|---|---|
| mXCR1 | 11.7 | 7.3 |

### Example 3

The methods in this example were carried out as described in Example 1.

### mXCR1 is expressed in the CD4+CD25+ regulatory T cells

The CD4+CD25+ T cell population is the most accepted naturally occurring form of regulatory T cells (Ramsdell F. Foxp3 and natural regulatory T cells: key to a cell lineage? Immunity. 2003: 19(2); 165-8). CD4+CD25+, CD4+CD25- and CD8+ were sorted from C3H/HeJ mice, RNA was isolated and RT-PCR was performed. Surprisingly, high levels of mXCR1 were detected within the CD4+CD25+ population. This finding further indicates that mXCR1 is a marker for tolerant or regulatory T cells.

### Mouse and human XCR1 contain major homologous sequences

The cDNA sequences of mouse and human XCR1 (Genebank designation numbers NM_011798 and AY275469) were compared using the DNASTAR MegAlign software (GATC Biotech AG, Konstanz, Germany; Martinez/Needle-man method). The similarity index was 74.8 % with minor gaps. No splice variants are described for the mouse or human sequences. Further XCR1 cDNA and protein sequences are known for rat and pig (Genebank designation numbers XP_236740 and NM_001001622).

## Claims

1. A method for measuring the tolerance status of a T cell sample from a patient suffering from a transplant rejection reaction or graft-versus-host disease, comprising the steps of
a) measuring the expression level of XCR1 in the T cell sample,
b) comparing the measured expression level with a reference value, said reference value being representative of the expression level of XCR1 in a control T cell sample.

2. The method according to claim 1, wherein
c) if the measured expression level is lower than the reference value, then it indicates that the T cell(s) of the sample are less tolerant than the T cells of the control T cell sample.

3. The method according to claim 1, wherein
c) if the measured expression level is higher than the reference value, then it indicates that the T cell(s) of the sample are more tolerant than the T cells of the control T cell sample.

4. The method according any of claims 1 to 3, wherein the expression level of XCR1 of particular T cell subpopulation in the T cell sample is measured and wherein the reference value is representative of the expression level of XCR1 in a corresponding T cell subpopulation in the control T cell sample.

5. The method according to claim 5, wherein the particular T cell subpopulation is chosen from the group consisting of
i) cytotoxic T cells,
ii) helper T cells,
iii) regulatory T cells,
iv) CD8+T cells
v) CD4+ T cells
vi) CD4+ CD25+T cells
vii) CD4+ CD25- Tcells
viii) a subpopulation having a T-cell receptor subtype directed against a particular MHC or HLA subtype.
ix) a subpopulation having a T-cell receptor subtype directed against a particular antigen.

6. Use of the expression level of XCR1 for measuring the tolerance status of a T cell sample from a patient suffering from a transplant rejection reaction or graft-versus-host-disease.

7. A method for diagnosing or monitoring of an immune response in a patient suffering from a transplant rejection reaction or graft-versus-host disease, comprising the steps of
a) measuring the expression level of XCR1 in the T cell sample,
b) comparing the measured expression level with a reference value, said reference value being representative of the expression level of XCR1 in a T cell sample taken in a known state of the immune response, wherein
c) if the measured expression level is lower than the reference value, then it indicates that the immune response is stronger compared to the known state, or
d) if the measured expression level is higher than the reference value, then it indicates that the immune response is weaker compared to the known state.

8. Use of the expression level of XCR1 in a T cell sample from a patient suffering from a transplant rejection reaction or graft-versus-host disease for diagnosing or monitoring of an immune response.

9. A method for diagnosing or monitoring of a transplant rejection reaction or graft-versus-host disease, comprising the steps of
a) measuring the expression level of XCR1 in the T cell sample,
b) comparing the measured expression level with a reference value, said reference value being representative of the expression level of XCR1 in a T cell sample taken in a known state of the disease, wherein
c) if the measured expression level is lower than the reference value, then it indicates that the disease is more severe compared to the known state, or
d) if the measured expression level is higher than the reference value, then it indicates that the disease is less severe compared to the known state.

10. Use of the expression level of XCR1 in a T cell sample for diagnosing or monitoring of a transplant rejection reaction or graft-versus-host disease.

11. An in vitro method for determining the required stringency of immunosuppression in a subject suffering from a transplant rejection reaction or graft-versus-host disease to be treated with immunosuppression, comprising the steps of
a) measuring the expression level of XCR1 in a T cell sample of a subject,
b) comparing the measured expression level to a reference value, said reference value being representative of the expression level of XCR1 in a T cell sample from a control subject requiring a known stringency of immunosuppression,
wherein
c) if the measured expression level is lower than the reference value, then it indicates that the stringency of immunosuppression should be maintained at the current level or increased compared to the stringency required by the control subject, or
d) if the measured expression level is higher than the reference value, then it indicates that the stringency of immunosuppression may be lowered compared to the stringency required by the control subject.

12. An in vitro method for determining the required stringency of immunosuppression in a subject suffering from a transplant rejection reaction or graft-versus-host disease who is already treated by immunosuppression, comprising the steps of
a) measuring the expression level of XCR1 in a T cell sample of the subject,
b) comparing the measured expression level to the expression level of XCR1 in a control T cell sample obtained from the subject at an earlier time, wherein
c) if the measured expression level is lower than the expression level in the control T cell sample, then it indicates that the stringency of immunosuppression should be maintained at the current level or increased compared to the stringency at the earlier time, or
d) if the measured expression level is higher than the expression level in the control T cell sample, then it indicates that the stringency of immunosuppression may be lowered compared to the stringency at the earlier time.

13. Use of the expression level of XCR1 in a T cell sample of a subject from a patient suffering from a transplant rejection reaction or graft-versus-host disease for determining the required stringency of immunosuppression in said subject.

14. Use of the primers as defined by SEQ ID NO: 5 and SEQ ID NO: 6 for measuring the expression level of XCR1 in a T cell sample of a subject suffering from a transplant rejection reaction or graft-versus-host disease for determining the required stringency of immunosuppression in said subject.

15. An in vitro method to identify or validate an inhibitor or inducer of T cell tolerance in transplant rejection reactions or graft-versus-host disease, comprising the steps of
a) providing a T cell,
b) bringing the T cell in contact with a candidate for an inhibitor or inducer,
c) measuring the expression level of XCR1 in the T cell of step b), and
d) comparing the measured expression level with the expression level of XCR1 in the T cell before step b), or
e) comparing the expression level with expression level of XCR1 in a corresponding T cell not brought in contact with the candidate for an inhibitor or inducer.

16. A method to identify or validate an inhibitor or inducer of T cell tolerance or immune system tolerance in transplant rejection reactions or graft-versus-host disease, comprising the steps of
a) providing a non-human animal,
b) exposing the non-human animal to a candidate for an inhibitor or inducer,
c) measuring, preferably in vitro, the expression level of XCR1 in a T cell sample obtained from the subject after step b), and
d) comparing the measured expression level with the expression level of XCR1 in a T cell sample obtained from the non-human animal before step b), or
e) comparing the measured expression level with the expression level of XCR1 in a corresponding T cell sample obtained from a corresponding subject not brought in contact with the candidate for an inhibitor or inducer
wherein methods of treatment of the animal body are to be excluded.

## Patentansprüche

1. Verfahren zum Messen des Toleranzstatus von einer T-Zellprobe eines Patienten, der an einer Transplantatabstoßungsreaktion oder an einer Graft-versus-Host-Erkrankung leidet, umfassen die Schritte
a) Messen des Expressionsspiegels von XCR1 in einer T-Zellprobe,
b) Vergleichen des gemessenen Expressionsspiegels mit einem Referenzwert, wobei dieser Referenzwert repräsentativ für den Expressionsspiegel für XCR1 in einer Kontroll-T-Zellprobe ist.

2. Verfahren nach Anspruch 1, wobei
c) wenn der gemessene Expressionsspiegel geringer als der Referenzwert ist, dies anzeigt, dass die T-Zellen der Probe weniger tolerant sind als die T-Zellen der Kontroll-T-Zellprobe.

3. Verfahren nach Anspruch 1, wobei
c) wenn der gemessene Expressionsspiegel größer als der Referenzwert ist, dies anzeigt, dass die T-Zellen der Probe stärker tolerant sind, als die T-Zellen der Kontroll-T-Zellprobe.

4. Verfahren nach einem der Ansprüche 1 bis 3, wobei der Expressionsspiegel von XCR1 einer bestimmten T-Zell-Subpopulation in der T-Zellprobe gemessen wird und wobei der Referenzwert repräsentativ für den Expressionsspiegel von XCR1 in einer entsprechenden T-Zell-Subpopulation in der Kontroll-T-Zellprobe ist.

5. Verfahren nach Anspruch 5, wobei die bestimmte T-Zell-Subpopulation ausgewählt ist aus der Gruppe bestehend aus:
i) cytotoxischen T-Zellen,
ii) Helfer-T-Zellen,
iii) regulatorischen T-Zellen,
iv) CD8+ T-Zellen,
v) CD4+ T-Zellen,
vi) CD4+CD25+T-Zellen,
vii) CD4+CD25- T-Zellen,
viii) eine Subpopulation, die einen T-Zellrezeptorsubtyp aufweist, der gegen einen bestimmten MHC oder HLA-Subtyp gerichtet ist,
ix) eine Subpopulation, die einen T-Zellrezeptorsubtyp aufweist, der gegen ein bestimmtes Antigen gerichtet ist.

6. Verwendung des Expressionsspiegels von XCR1 für die Messung des Toleranzstatus einer T-Zellprobe eines Patienten, der an einer Transplantatabstoßungsreaktion oder an Graft-versus-Host-Erkrankung leidet.

7. Verfahren zur Diagnose oder zur Überwachung einer Immunantwort in einem Patienten, der an einer Transplantatabstoßungsreaktion oder an Graft-versus-Host-Erkrankung leidet, umfassend die Schritte
a) Messen des Expressionsspiegel von XCR1 in einer T-Zellprobe,
b) Vergleichen des gemessenen Expressionsspiegels mit einem Referenzwert, wobei der Referenzwert repräsentativ für den Expressionsspiegel von XCR1 in einer T-Zellprobe ist, die bei einem bekannten Status der Immunantwort genommen wurde, wobei
c) wenn der gemessene Expressionsspiegel geringer als der Referenzwert ist, dies anzeigt, dass die Immunantwort stärker im Vergleich zu dem bekannten Status ist oder
d) wenn der gemessene Expressionsspiegel höher als der Referenzwert ist, dies anzeigt, dass die Immunantwort schwächer als bei dem bekannten Status ist.

8. Verwendung des Expressionsspiegels von XCR1 in einer T-Zellprobe eines Patienten, der an einer Transplantatabstoßungsreaktion oder an Graft-versus-Host-Erkrankung leidet, zur Diagnose oder zum Überwachen einer Immunantwort.

9. Verfahren zur Diagnose oder zur Überwachung von Transplantatabstoßungsreaktionen oder an Graft-versus-Host-Erkrankungen, umfassend die Schritte
a) Messen des Expressionsspiegels von XCR1 in einer T-Zellprobe,
b) Vergleichen des gemessenen Expressionsspiegels mit einem Referenzwert, wobei der Referenzwert repräsentativ für den Expressionsspiegel von XCR1 in einer T-Zellprobe ist, die bei einem bekannten Status der Erkrankung genommen wurde, wobei
c) wenn der gemessene Expressionsspiegel geringer als der Referenzwert ist, dies anzeigt, dass die Erkrankung stärker ausgeprägt ist im Vergleich zu dem bekannten Status oder
d) wenn der gemessene Expressionsspiegel höher als der Referenzwert ist, dies anzeigt, dass die Erkrankung weniger stark ausgeprägt ist verglichen mit dem bekannten Status.

10. Verwendung des Expressionsspiegels von XCR1 in einer T-Zellprobe zur Diagnose oder zum Überwachen von Transplantatabstoßungsreaktionen oder einer Graft-versus-Host-Erkrankung.

11. In vitro-Verfahren zur Bestimmung der benötigten Stringenz der Immunsuppression in einem Subjekt, das an Transplantatabstoßungsreaktionen oder Graft-versus-Host-Erkrankung leidet und mittels Immunsuppression behandelt werden soll, umfassend die Schritte
a) Messen des Expressionsspiegels von XCR1 in einer T-Zellprobe des Subjekts,
b) Vergleichen des gemessenen Expressionsspiegels mit einem Referenzwert, wobei der Referenzwert repräsentativ für den Expressionsspiegel von XCR1 in einer T-Zellprobe aus einem Kontrollsubjekt sein soll, das Immunsuppression von bekannter Stringenz benötigt, wobei
c) wenn der gemessene Expressionsspiegel geringer als der Referenzwert ist, dies bedeutet, dass die Stringenz der Immunsuppression bei dem bestehenden Maß erhalten werden oder verstärkt werden soll verglichen mit der Stringenz, die bei dem Kontrollsubjekt benötigt wird oder
d) wenn der gemessene Expressionsspiegel höher als der Referenzwert ist, dies bedeutet, dass die Stringenz der Immunsuppression verglichen mit der für ein Kontrollsubjekt benötigte Stringenz verringert werden kann.

12. In vitro-Verfahren zur Bestimmung der benötigen Stringenz der Immunsuppression bei einem Subjekt, das an Transplantatabstoßungsreaktion oder Graft-versus-Host-Erkrankung leidet und bereits mittels Immunsuppression behandelt wird, umfassend die Schritte
a) Messen des Expressionsspiegels von XCR1 in einer T-Zellprobe des Subjekts,
b) Vergleichen des gemessenen Expressionsspiegels mit einem Expressionsspiegel für XCR1 in einer Kontroll-T-Zellprobe, erhalten von dem Subjekt zu einem früheren Zeitpunkt, wobei
c) wenn der gemessene Expressionsspiegel geringer als der Expressionsspiegel in der Kontroll-T-Zellprobe ist, dies bedeutet, dass die Stringenz der Immunsuppression auf dem bestehenden Maß erhalten werden oder verstärkt werden soll verglichen mit der Stringenz zu einem früheren Zeitpunkt oder
d) wenn der gemessene Expressionsspiegel höher als der Expressionsspiegel in einer Kontroll-T-Zellprobe ist, dies bedeutet, dass die Stringenz der Immunsuppression verringert werden soll verglichen mit der Stringenz zu einem früheren Zeitpunkt.

13. Verwendung des Expressionsspiegels von XCR1 in einer T-Zellprobe eines Subjektes eines Patienten, der an einer Transplantatabstoßungsreaktion oder Graft-versus-Host-Erkrankung leidet, zur Bestimmung der benötigten Stringenz der Immunsuppression in dem Subjekt.

14. Verwendung eines Primers, wie durch SEQ ID Nos 5 und 6 definiert, zum Messen des Expressionsspiegels von XCR1 in einer T-Zellprobe eines Subjektes, das an Transplantatabstoßungsreaktion oder Graft-versus-Host-Erkrankung leidet, zur Bestimmung der benötigten Stringenz der Immunsuppression in dem Subjekt.

15. In-vitro-Verfahren zur Bestimmung oder Validierung eines Inhibitors oder Induzierers von T-Zelltoleranz bei Transplantatabstoßungsreaktion oder Graft-versus-Host-Erkrankung, umfassend die Schritte
a) Bereitstellen einer T-Zelle,
b) in Kontakt bringen der T-Zelle mit einem Kandidaten für einen Inhibitor oder Induzierer,
c) Messen des Expressionsspiegels von XCR1 in der T-Zelle gemäß Schritt b) und
d) Vergleichen des gemessenen Expressionsspiegels mit dem Expressionsspiegels von XCR1 in der T-Zelle vor Schritt b) oder
e) Vergleichen des Expressionsspiegels mit dem Expressionsspiegel für XCR1 in einer entsprechenden T-Zelle, die nicht in Kontakt mit dem Kandidaten für den Inhibitor oder Induzierer gebracht wurde.

16. Verfahren zur Identifizierung oder Validierung eines Inhibitors oder Induzierers von T-Zelltoleranz oder Immunsystemtoleranz in Transplantatabstoßungsreaktionen oder bei Graft-versus-Host-Erkrankungen, umfassend die Schritte
a) Bereitstellen eines nicht-menschlichen Tieres,
b) Aussetzen des nicht-menschlichen Tieres einem Kandidaten für einen Inhibitor oder Induzierer,
c) Messen, vorzugsweise in vitro, des Expressionsspiegels von XCR1 in einer T-Zellprobe, die von dem Subjekt nach Schritt b) erhalten wurde und
d) Vergleichen des gemessenen Expressionsspiegels mit dem Expressionsspiegel von XCR1 in einer T-Zellprobe, erhalten von einem nicht-menschlichem Tier vor Schritt b) oder
e) Vergleichen des gemessenen Expressionsspiegels mit dem Expressionsspiegel von XCR1 in einer entsprechenden T-Zellprobe, erhalten von einem entsprechenden Subjekt, das nicht in Kontakt mit dem Kandidaten für den Inhibitor oder Induzierer gebracht wurde, wobei Verfahren zur Behandlung des tierischen Körpers ausgeschlossen sind.

## Revendications

1. Procédé pour mesurer l'état de tolérance d'un échantillon de lymphocytes T provenant d'un patient souffrant d'une réaction de rejet de transplantation ou de la maladie du greffon contre l'hôte, comprenant les étapes consistant à :
a) mesurer le niveau d'expression de XCR1 dans l'échantillon de lymphocytes T,
b) comparer le niveau d'expression mesuré à une valeur de référence, ladite valeur de référence étant représentative du niveau d'expression de XCR1 dans un échantillon de lymphocytes T témoin.

2. Procédé selon la revendication 1, dans lequel :
c) si le niveau d'expression mesuré est inférieur à la valeur de référence, cela indique que le ou les lymphocytes T de l'échantillon sont moins tolérants que les lymphocytes T de l'échantillon de lymphocytes T témoin.

3. Procédé selon la revendication 1, dans lequel :
c) si le niveau d'expression mesuré est supérieur à la valeur de référence, cela indique que le ou les lymphocytes T de l'échantillon sont plus tolérants que les lymphocytes T de l'échantillon de lymphocytes T témoin.

4. Procédé selon l'une quelconque des revendications 1 à 3, dans lequel on mesure le niveau d'expression de XCR1 d'une sous-population particulière de lymphocytes T dans l'échantillon de lymphocytes T, et dans lequel la valeur de référence est représentative du niveau d'expression de XCR1 dans une sous-population de lymphocytes T correspondante dans l'échantillon de lymphocytes T témoin.

5. Procédé selon la revendication 5, dans lequel la sous-population particulière de lymphocytes T est choisie dans le groupe constitué par :
i) des lymphocytes T cytotoxiques,
ii) des lymphocytes T auxiliaires,
iii) des lymphocytes T régulateurs,
iv) des lymphocytes T de type CD8+
v) des lymphocytes T de type CD4+
vi) des lymphocytes T de type CD4+ CD25+
vii) des lymphocytes T de type CD4+ CD25-
viii) une sous-population ayant un sous-type de récepteur de lymphocytes T dirigé contre un sous-type particulier de CMH ou HLA.
ix) une sous-population présentant un sous-type de récepteur de lymphocytes T dirigé contre un antigène particulier.

6. Utilisation du niveau d'expression de XCR1 pour mesurer l'état de tolérance d'un échantillon de lymphocytes T provenant d'un patient souffrant d'une réaction de rejet de transplantation ou de la maladie du greffon contre l'hôte.

7. Procédé pour le diagnostic ou la surveillance d'une réponse immunitaire chez un patient souffrant d'une réaction de rejet de transplantation ou de la maladie du greffon contre l'hôte, comprenant les étapes consistant à :
a) mesurer le niveau d'expression de XCR1 dans l'échantillon de lymphocytes T,
b) comparer le niveau d'expression mesuré à une valeur de référence, ladite valeur de référence étant représentative du niveau d'expression de XCR1 dans un échantillon de lymphocytes T prélevés à un état connu de la réponse immunitaire, dans lequel
c) si le niveau d'expression mesuré est inférieur à la valeur de référence, cela indique que la réponse immunitaire est plus forte en comparaison de celle de l'état connu, ou
d) si le niveau d'expression mesuré est supérieur à la valeur de référence, cela indique que la réponse immunitaire est plus faible en comparaison de celle de l'état connu.

8. Utilisation du niveau d'expression de XCR1 dans un échantillon de lymphocytes T provenant d'un patient souffrant d'une réaction de rejet de transplantation ou de la maladie du greffon contre l'hôte dans le but de diagnostiquer ou de surveiller une réponse immunitaire.

9. Procédé pour le diagnostic ou la surveillance d'une réaction de rejet de transplantation ou de la maladie du greffon contre de l'hôte, comprenant les étapes consistant à :
a) mesurer le niveau d'expression de XCR1 dans l'échantillon de lymphocytes T,
b) comparer le niveau d'expression mesuré à une valeur de référence, ladite valeur de référence étant représentative du niveau d'expression de XCR1 dans un échantillon de lymphocytes T prélevé à un état connu de la maladie, dans lequel :
c) si le niveau d'expression mesuré est inférieur à la valeur de référence, cela indique que la maladie est plus grave en comparaison de celle à l'état connu, ou
d) si le niveau d'expression mesuré est supérieur à la valeur de référence, cela indique que la maladie est moins grave en comparaison de celle à l'état connu.

10. Utilisation du niveau d'expression de XCR1 dans un échantillon de lymphocytes T dans le but de diagnostiquer ou de surveiller une réaction de rejet de transplantation ou la maladie du greffon contre l'hôte.

11. Procédé in vitro pour déterminer la stringence requise d'une immunosuppression chez un sujet souffrant d'une réaction de rejet de transplantation ou de la maladie du greffon contre l'hôte, que l'on souhaite traiter par immunosuppression, comprenant les étapes consistant à :
a) mesurer le niveau d'expression de XCR1 dans un échantillon de lymphocytes T d'un sujet,
b) comparer le niveau d'expression mesuré à une valeur de référence, ladite valeur de référence étant représentative du niveau d'expression de XCR1 dans un échantillon de lymphocytes T prélevé chez un sujet témoin nécessitant une stringence d'immunosuppression connue, dans lequel :
c) si le niveau d'expression mesuré est inférieur à la valeur de référence, cela indique que la stringence d'immunosuppression devrait être maintenue au niveau actuel ou augmentée en comparaison de la stringence requise par le sujet témoin, ou
d) si le niveau d'expression mesuré est supérieur à la valeur de référence, cela indique alors que la stringence d'immunosuppression peut être réduite en comparaison de la stringence requise par le sujet témoin.

12. Procédé in vitro pour déterminer la stringence requise d'une immunosuppression chez un sujet souffrant d'une réaction de rejet de transplantation ou de la maladie du greffon contre l'hôte, le sujet en question étant déjà traité par immunosuppression, comprenant les étapes consistant à :
a) mesurer le niveau d'expression de XCR1 dans un échantillon de lymphocytes T du sujet,
b) comparer le niveau d'expression mesuré au niveau d'expression de XCR1 dans un échantillon de lymphocytes T témoin prélevé chez le sujet à un stade plus précoce dans le temps, dans lequel :
c) si le niveau d'expression mesuré est inférieur au niveau d'expression dans l'échantillon de lymphocytes T témoin, cela indique alors que la stringence de l'immunosuppression devrait être maintenue au niveau actuel ou augmentée en comparaison de la stringence déterminée à un stade plus précoce dans le temps, ou
d) si le niveau d'expression mesuré est supérieur au niveau d'expression dans l'échantillon de lymphocytes T témoin, cela indique alors que la stringence de l'immunosuppression peut être réduite en comparaison de la stringence correspondant à un stade plus précoce dans le temps.

13. Utilisation du niveau d'expression de XCR1 dans un échantillon de lymphocytes T prélevé chez un sujet souffrant d'une réaction de rejet de transplantation ou de la maladie du greffon contre l'hôte, dans le but de déterminer la stringence requise de l'immunosuppression chez ledit sujet.

14. Utilisation des amorces, telles que définies par les séquences représentées par la SEQ ID N° 5 et par la SEQ ID N° 6, pour mesurer le niveau d'expression de XCR1 dans un échantillon de lymphocytes T chez un sujet souffrant d'une réaction de rejet de transplantation ou de la maladie du greffon contre l'hôte, afin de déterminer la stringence requise de l'immunosuppression chez ledit sujet.

15. Procédé in vitro pour identifier ou valider un inhibiteur ou un inducteur de tolérance de lymphocytes T dans le cadre de réactions de rejet de transplantation ou de la maladie du greffon contre l'hôte, comprenant les étapes consistant à :
a) mettre en oeuvre un lymphocyte T,
b) mettre le lymphocyte T en contact avec un candidat pour un inhibiteur ou pour un inducteur,
c) mesurer le niveau d'expression de XCR1 dans le lymphocyte T obtenu à l'étape b), et
d) comparer le niveau d'expression mesuré avec le niveau d'expression de XCR1 dans le lymphocyte T avant l'étape b), ou
e) comparer le niveau d'expression au niveau d'expression de XCR1 dans un lymphocyte T correspondant qui n'a pas été mis en contact avec le candidat pour un inhibiteur ou pour un inducteur.

16. Procédé pour identifier ou valider un inhibiteur ou un inducteur de tolérance de lymphocytes T ou de tolérance du système immunitaire dans le cadre de réactions de rejet de transplantation ou de la maladie du greffon contre l'hôte, comprenant les étapes consistant à :
a) mettre en oeuvre un animal non de type humain,
b) exposer l'animal non de type humain à un candidat pour un inhibiteur ou pour un inducteur,
c) mesurer, de préférence in vitro, le niveau d'expression de XCR1 dans un échantillon de lymphocytes T prélevé chez le sujet après l'étape b), et
d) comparer le niveau d'expression mesuré au niveau d'expression de XCR1 dans un échantillon de lymphocytes T provenant d'un animal non de type humain avant l'étape b), ou
e) comparer le niveau d'expression mesuré au niveau d'expression de XCR1 dans un échantillon de lymphocytes T correspondant provenant d'un sujet correspondant qui n'a pas été mis en contact avec le candidat pour un inhibiteur ou pour un inducteur
les procédés de traitement du corps de l'animal doivent être, ici, exclus.


### SEQUENCE LISTING

<110> Deutsches Krebsforschungszentrum, Stiftung des öffentlichen Rechts Ruprecht-Karls-Universität Heildelberg
<120> Use of XCR1 for diagnosing or monitoring of immune tolerance
<130> DK62911PC
<150> EP 05004392.6
   <151> 2005-03-01
<160> 8
<170> PatentIn version 3.1
<210> 1
   <211> 322
   <212> PRT
   <213> Mus musculus
<400> 1
   Met Glu Ser Ser Thr Ala Phe Tyr Asp Tyr His Asp Lys Leu Ser Leu
   1               5                   10                  15
   Leu Cys Glu Asn Asn Val Ile Phe Phe Ser Thr Ile Ser Thr Ile Val
               20                  25                  30
   Leu Tyr Ser Leu Val Phe Leu Leu Ser Leu Val Gly Asn Ser Leu Val
           35                  40                  45
   Leu Trp Val Leu Val Lys Tyr Glu Asn Leu Glu Ser Leu Thr Asn Ile
       50                  55                  60
   Phe Ile Leu Asn Leu Cys Leu Ser Asp Leu Met Phe Ser Cys Leu Leu
   65                  70                  75                  80
   Pro Val Leu Ile Ser Ala Gln Trp Ser Trp Phe Leu Gly Asp Phe Phe
                   85                  90                  95
   Cys Lys Phe Phe Asn Met Ile Phe Gly Ile Ser Leu Tyr Ser Ser Ile
               100                 105                 110
   Phe Phe Leu Thr Ile Met Thr Ile His Arg Tyr Leu Ser Val Val Ser
           115                 120                 125
   Pro Ile Ser Thr Leu Gly Ile His Thr Leu Arg Cys Arg Val Leu Val
       130                 135                 140
   Thr Ser Cys Val Trp Ala Ala Ser Ile Leu Phe Ser Ile Pro Asp Ala
   145                 150                 155                 160
   Val Phe His Lys Val Ile Ser Leu Asn Cys Lys Tyr Ser Glu His His
                   165                 170                 175
   Gly Phe Leu Ala Ser Val Tyr Gln His Asn Ile Phe Phe Leu Leu Ser
               180                 185                 190
   Met Gly Ile Ile Leu Phe Cys Tyr Val Gln Ile Leu Arg Thr Leu Phe
           195                 200                 205
   Arg Thr Arg Ser Arg Gln Arg His Arg Thr Val Arg Leu Ile Phe Thr
       210                 215                 220
   Val Val Val Ala Tyr Phe Leu Ser Trp Ala Pro Tyr Asn Leu Thr Leu
   225                 230                 235                 240
   Phe Leu Lys Thr Gly Ile Ile Gln Gln Ser Cys Glu Ser Leu Gln Gln
                   245                 250                 255
   Leu Asp Ile Ala Met Ile Ile Cys Arg His Leu Ala Phe Ser His Cys
               260                 265                 270
   Cys Phe Asn Pro Val Leu Tyr Val Phe Val Gly Ile Lys Phe Arg Arg
           275                 280                 285
   His Leu Lys His Leu Phe Gln Gln Val Trp Leu Cys Arg Lys Thr Ser
       290                 295                 300
   Ser Thr Val Pro Cys Ser Pro Gly Thr Phe Thr Tyr Glu Gly Pro Ser
   305                 310                 315                 320
   Phe Tyr
<210> 2
   <211> 333
   <212> PRT
   <213> Homo sapiens
<400> 2 <210> 3
   <211> 333
   <212> PRT
   <213> Sus scrofta
<400> 3 <210> 4
   <211> 322
   <212> PRT
   <213> Rattus norvegicus
<400> 4
<210> 5
   <211> 22 <212> DNA
   <213>Artificial sequence
<220>
<223> Primer mXCR1 FP
<400> 5
   gtccagacag agacaccgaa ca 22
<210> 6
   <211> 20
   <212> DNA
   <213> artificial sequence
<220>
<223> Primer mXCR1 RP
<400> 6
   gcccagctga ggaagtatgc 20
<210> 7
   <211> 25
   <212> DNA
   <213> artificial sequence
<220>
<223> Primer HPRT FP
<400> 7
   acacctgcta attttactgg caaca 25
<210> 8
   <211> 24
   <212> DNA
   <213> artificial sequence
<220>
<223> Primer HPRT RP
<400> 8
   tggaaaagcc aaatacaaag ccta 24